# EUROPEAN PATENT SPECIFICATION

(11) **EP 3 840 754 B1**
(45) Date of publication and mention of the grant of the patent: **18.11.2026**
(21) Application number: 19851623.9
(22) Date of filing: 23.08.2019
(51) Int. Cl.: C07D 221/18, C01B 21/096, C07C 53/128, C07C 53/16, C07C 55/12, C07C 57/30, C07C 57/44, C07C 59/06, C07C 65/05, C07C 229/64, C07C 307/02, C07C 309/05, C07C 309/35, A61P 25/16, A61K 31/473

(54) **ACID ADDITION SALTS OF APOMORPHINE, PHARMACEUTICAL COMPOSITIONS CONTAINING THE SAME, AND METHODS OF USING THE SAME**
SÄUREADDITIONSSALZE VON APOMORPHIN, ARZNEIMITTEL, DIE DIESE ENTHALTEN UND VERFAHREN ZU DEREN VERWENDUNG
SELS D'ADDITION D'ACIDES D'APOMORPHINE, COMPOSITIONS PHARMACEUTIQUES EN CONTENANT ET LEURS PROCÉDÉS D'UTILISATION

(30) Priority: 23.08.2018 US 201862721894 P
(43) Date of publication of application: 30.06.2021
(73) Proprietor: BIAL - Portela & CA, S.A., 4745-457 Coronado, S. Romão e S. Mamede (PT)
(72) Inventor: BILBAULT, Thierry, Cambridge, MA 02140 (US)
(74) Representative: Vossius & Partner Patentanwälte Rechtsanwälte mbB
(86) International application number: PCT/US2019/047876
(87) International publication number: WO 2020/041683

(56) References cited:
- WO-A1-90/12574
- WO-A1-90/12574
- WO-A2-2016/103262
- US-A1- 2010 316 712
- US-A1- 2013 287 854
- US-A1- 2018 133 146
- US-A1- 2018 133 146
- ANONYMOUS: "Select Committee on GRAS Substances (SCOGS) Opinion: Sulfamic acid (packaging)", GRAS SUBSTANCES (SCOGS) DATABASE, 10 June 2015 (2015-06-10), pages 1 - 1, XP055918392, Retrieved from the Internet <URL:http://wayback.archive-it.org/7993/20171031062009/https://www.fda.gov/Food/IngredientsPackagingLabeling/GRAS/SCOGS/ucm261478.htm> [retrieved on 20220505]
- "Handbook of pharmaceutical salts: properties, selection, and use", 2002, WILEY-VCH, Weinheim, ISBN: 978-3-906390-26-0, article STAHL; WERMUTH: "Cyclamic Acid", pages: 279 - 279, XP055918394

## Description

### FIELD

This disclosure relates to acid addition salts of apomorphine, solid crystalline forms thereof, pharmaceutical compositions containing them, and the use thereof, e.g., for the treatment of Parkinson's disease, restless leg syndrome, or sexual dysfunction.

### BACKGROUND

Parkinson's disease (PD) affects more than 1.5 million individuals in the United States. The symptoms of PD vary from patient to patient. The common primary symptoms are a paucity of movement and rigidity, characterized by an increased stiffness of voluntary skeletal muscles. Additional symptoms include resting tremor, slowness of movement (bradykinesia), poor balance, and walking problems. Common secondary symptoms include depression, sleep disturbance, dizziness, stooped posture, dementia, problems with speech, breathing, and swallowing. These symptoms become progressively worse with time, ultimately resulting in death.
US 2018/0133146 refers inter alia to a method of treating an "off" episode in a subject having Parkinson's disease, said method comprising: (a) providing a film having a first portion comprising apomorphine particles comprising an acid addition salt of apomorphine and a second portion comprising a pH neutralizing agent; and (b) sublingually administering said film to said subject, wherein said film comprises an acid addition salt of apomorphine in an amount sufficient to produce, on average, following administration to the subject: (i) an apomorphine plasma concentration of at least 2.64 ng/ml within 30 minutes, and (ii) an apomorphine Cmax is less than 10 ng/mL.
WO 90/12574 discloses dopamine agonist compounds which are aporphine compounds. The compounds have a dioxy group on an aromatic nucleus which is cleaved in vivo to release compounds with two adjacent hydroxy groups on the aromatic nucleus.

Apomorphine hydrochloride formulated for sublingual administration is useful for treating patients with Parkinson's disease. However, there remains a need for identifying new acid addition salts of apomorphine that exhibit improved physical properties (e.g., aqueous solubility and substantially neutral pH in aqueous environments) suitable for use in pharmaceutical compositions.

### SUMMARY

The present invention relates to an acid addition salt of apomorphine, wherein the acid is sulfamic acid.

Any reference to the acid addition salts of the following acids in the present specification is for reference purposes only: glycolic acid, isobutyric acid, 2,2-dichloroacetic acid, 4-acetamidobenzoic acid, 4-aminosalicylic acid, cinnamic acid, cyclamic acid, ethane disulfonic acid, gentisic acid, glutaric acid, methylbenzoic acid, or 1,5-naphthalene disulfonic acid.

In some embodiments, the acid addition salt of apomorphine may be used as an active agent in pharmaceutical compositions (e.g., unit dosage forms). In some embodiments, disclosed are solid crystalline forms of acid addition salts of apomorphine.

In some embodiments, the solid crystalline form of apomorphine glycolate has an X-ray powder diffraction (XRPD) pattern comprising one or more peaks at a diffraction angle 2θ (°) selected from the group consisting of 10.3+0.2, 12.4±0.2, 16.1±0.2, 21.3±0.2, 21.7±0.2, and 23.7±0.2. In some embodiments, the X-ray powder diffraction (XRPD) pattern comprises a peak at a diffraction angle 2θ (°) of 10.3±0.2. In some embodiments, the X-ray powder diffraction (XRPD) pattern comprises a peak at a diffraction angle 2θ (°) of 12.4±0.2. In some embodiments, the X-ray powder diffraction (XRPD) pattern comprises a peak at a diffraction angle 2θ (°) of 16.1±0.2. In some embodiments, the X-ray powder diffraction (XRPD) pattern comprises a peak at a diffraction angle 2θ (°) of 21.3±0.2. In some embodiments, the X-ray powder diffraction (XRPD) pattern comprises a peak at a diffraction angle 2θ (°) of 21.7+0.2. In some embodiments, the X-ray powder diffraction (XRPD) pattern comprises a peak at a diffraction angle 2θ (°) of 23.7±0.2.

In some embodiments, the solid crystalline form of apomorphine glycolate has an XRPD pattern comprising peaks at diffraction angles 2θ (°) of 10.3+0.2, 12.4±0.2, 16.1±0.2, 16.3±0.2, 20.6±0.2, 21.3±0.2, 21.7±0.2, 23.3±0.2, 24.7±0.2, and 23.7±0.2.

In some embodiments, the solid crystalline form of apomorphine glycolate has an X-ray powder diffraction (XRPD) pattern comprising peaks at diffraction angles 2θ (°) of 10.3+0.2, 12.4±0.2, 21.3±0.2, 21.7±0.2, and 23.7±0.2.

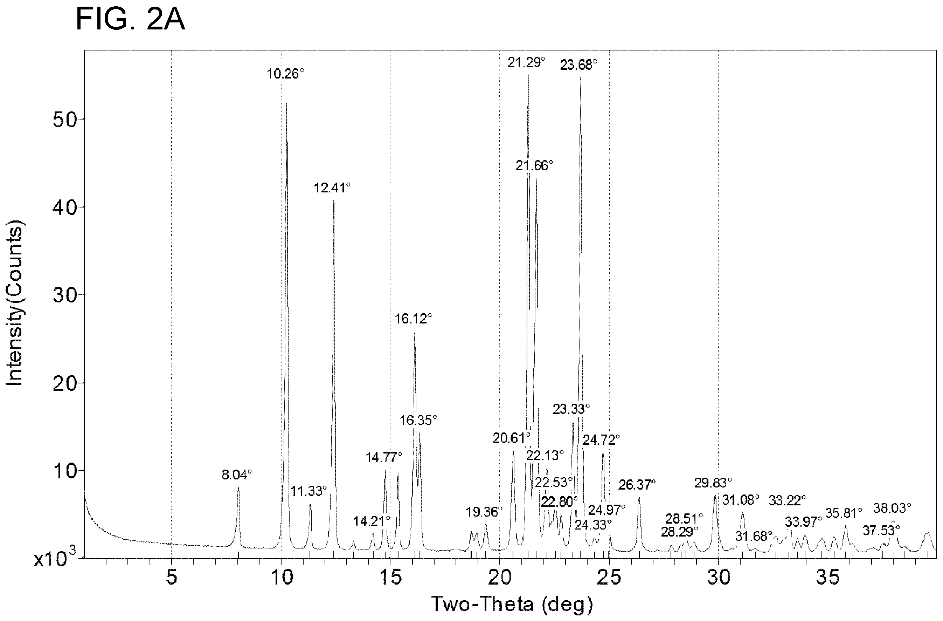

In some embodiments, the solid crystalline form of apomorphine glycolate has the X-ray powder diffraction (XRPD) pattern comprising peaks at diffraction angles 2θ (°) listed in Table 1. In some embodiments, the solid crystalline form of apomorphine glycolate is characterized by the X-ray powder diffraction (XRPD) pattern substantially as set forth in FIG. 2A.

In some embodiments, the solid crystalline form of apomorphine isobutyrate has an X-ray powder diffraction (XRPD) pattern comprising one or more peaks being at a diffraction angle 2θ (°) selected from the group consisting of 9.1±0.2, 11.6+0.2, 14.1±0.2, 16.1±0.2, 19.4±0.2, 21.5±0.2, 22.9±0.2, and 23.4±0.2. In some embodiments, the X-ray powder diffraction (XRPD) pattern comprises a peak at a diffraction angle 2θ (°) of 9.1±0.2. In some embodiments, the X-ray powder diffraction (XRPD) pattern comprises a peak at a diffraction angle 2θ (°) of 11.6+0.2. In some embodiments, the X-ray powder diffraction (XRPD) pattern comprises a peak at a diffraction angle 2θ (°) of 16.1±0.2. In some embodiments, the X-ray powder diffraction (XRPD) pattern comprises a peak at a diffraction angle 2θ (°) of 21.5±0.2. In some embodiments, the X-ray powder diffraction (XRPD) pattern comprises a peak at a diffraction angle 2θ (°) of 23.4±0.2. In some embodiments, the X-ray powder diffraction (XRPD) pattern comprises one or more peaks at diffraction angles 2θ (°) selected from the group consisting of 14.1±0.2, 19.4±0.2, and 22.9±0.2.

In some embodiments, the solid crystalline form of apomorphine isobutyrate has an X-ray powder diffraction (XRPD) pattern comprising peaks at diffraction angles 2θ (°) of 9.1+0.2, 11.6+0.2, 11.9±0.2, 13.2±0.2, 14.1±0.2, 16.1±0.2, 19.4±0.2, 21.6±0.2, 23.0±0.2, 23.4±0.2, and 24.7±0.2.

In some embodiments, the solid crystalline form of apomorphine isobutyrate has an X-ray powder diffraction (XRPD) pattern comprising peaks at diffraction angles 2θ (°) of 9.1±0.2, 14.1±0.2, 16.1±0.2, 21.6±0.2, 23.0±0.2, and 23.4±0.2.

In some embodiments, the solid crystalline form of apomorphine isobutyrate has an X-ray powder diffraction (XRPD) pattern comprising peaks at diffraction angles 2θ (°) of 9.1±0.2, 21.6+0.2, and 23.4±0.2.

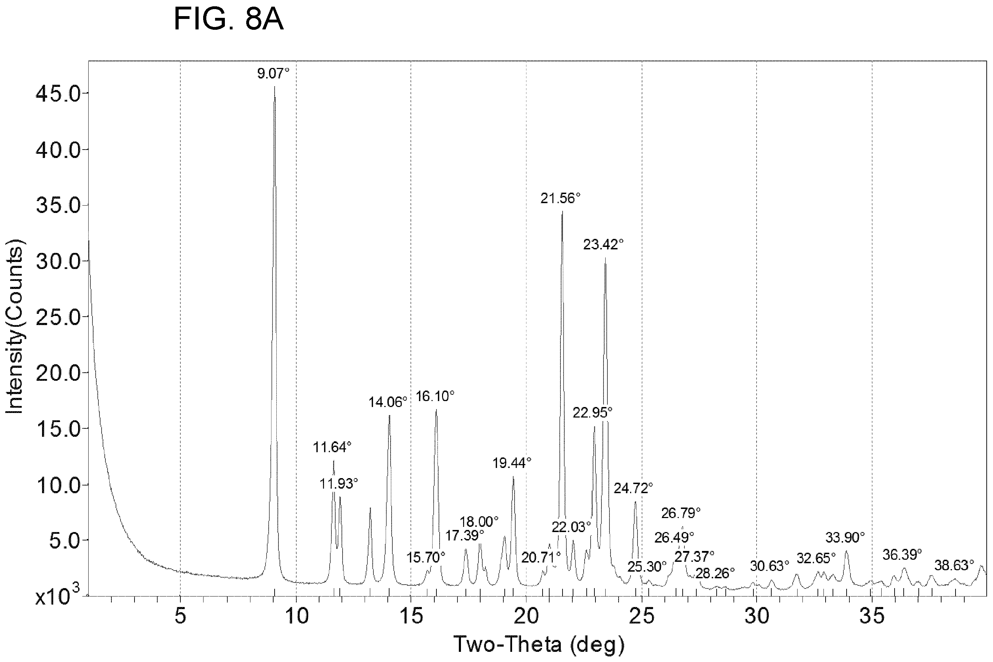

In some embodiments, the solid crystalline form of apomorphine isobutyrate has the X-ray powder diffraction (XRPD) pattern comprising peaks at diffraction angles 2θ (°) listed in Table 2. In some embodiments, the solid crystalline form of apomorphine isobutyrate is characterized by the X-ray powder diffraction (XRPD) pattern substantially as set forth in FIG. 8A.

In some embodiments, the solid crystalline form of apomorphine sulfamate has an X-ray powder diffraction (XRPD) pattern comprising peaks at diffraction angles 2θ (°) of 9.9±0.2, 11.7±0.2, 14.2±0.2, 14.8+0.2, 16.5±0.2, 18.6±0.2, 18.9±0.2, 21.7±0.2, 22.1±0.2, 22.4±0.2, 23.6±0.2, 23.9±0.2, 25.4±0.2, and 27.0±0.2. In some embodiments, the X-ray powder diffraction (XRPD) pattern comprises a peak at a diffraction angle 2θ (°) of 9.9±0.2. In some embodiments, the X-ray powder diffraction (XRPD) pattern comprises a peak at a diffraction angle 2θ (°) of 11.7+0.2. In some embodiments, the X-ray powder diffraction (XRPD) pattern comprises a peak at a diffraction angle 2θ (°) of 16.5±0.2. In some embodiments, the X-ray powder diffraction (XRPD) pattern comprises a peak at a diffraction angle 2θ (°) of 18.9±0.2. In some embodiments, the X-ray powder diffraction (XRPD) pattern comprises a peak at a diffraction angle 2θ (°) of 22.1±0.2. In some embodiments, the X-ray powder diffraction (XRPD) pattern comprises a peak at a diffraction angle 2θ (°) of 23.9±0.2. In some embodiments, the X-ray powder diffraction (XRPD) pattern comprises a peak at a diffraction angle 2θ (°) of 27.0±0.2. In some embodiments, the X-ray powder diffraction (XRPD) pattern comprises one or more peaks at diffraction angles 2θ (°) selected from the group consisting of 14.2+0.2, 14.8+0.2, 18.6±0.2, 20.0±0.2, 21.7±0.2, 22.4±0.2, 23.6±0.2, and 25.4±0.2.

In some embodiments, the solid crystalline form of apomorphine sulfamate has an X-ray powder diffraction (XRPD) pattern comprising peaks at diffraction angles 2θ (°) of 9.9±0.2, 11.7±0.2, 14.2±0.2, 14.8±0.2, 16.5±0.2, 18.6±0.2, 18.9±0.2, 21.7±0.2, 22.1±0.2, 22.4±0.2, 23.6±0.2, 23.9±0.2, 25.4±0.2, and 27.0±0.2.

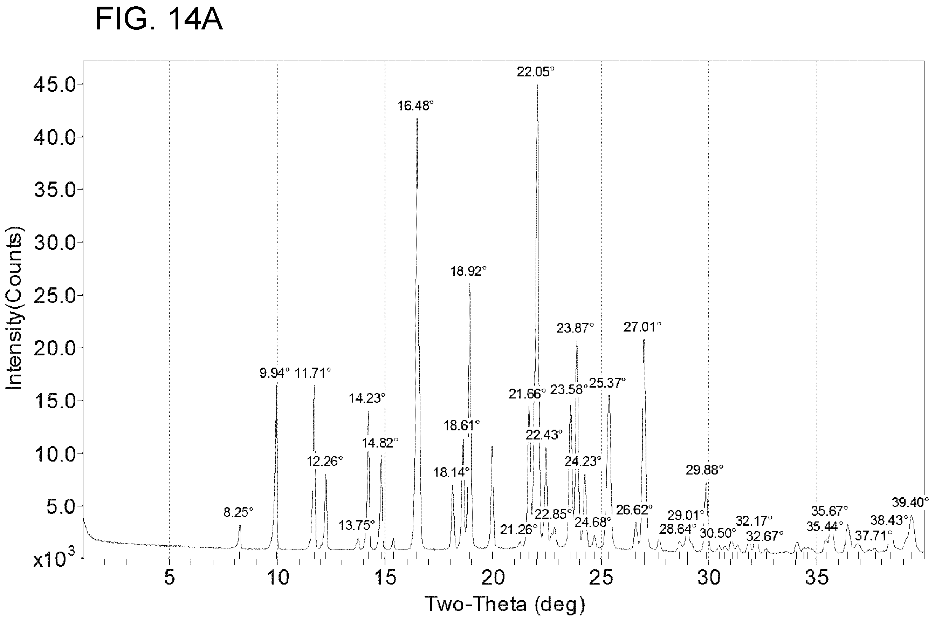

In some embodiments, the solid crystalline form of apomorphine sulfamate has the X-ray powder diffraction (XRPD) pattern comprising peaks at diffraction angles 2θ (°) listed in Table 3. In some embodiments, the solid crystalline form of apomorphine sulfamate is characterized by the X-ray powder diffraction (XRPD) pattern substantially as set forth in FIG. 14A.

In some embodiments, the solid crystalline form of apomorphine sulfamate has an X-ray powder diffraction (XRPD) pattern comprising peaks at diffraction angles 2θ (°) of 16.5±0.2, 18.6±0.2, 18.9±0.2, 22.1±0.2, 23.9±0.2, and 27.0±0.2.

In a related aspect, disclosed are pharmaceutical compositions comprising an acid addition salt of apomorphine or a crystalline salt form thereof and a pharmaceutically acceptable excipient. In some embodiments, the acid addition salt of apomorphine is enantiomerically enriched in an acid addition salt of (R)-apomorphine. In some embodiments, the enantiomeric excess for the acid addition salt of (R)-apomorphine is at least 90%, at least 95%, or at least 99%. In some embodiments, the pharmaceutical composition is in a unit dosage form. In some embodiments, the unit dosage form is a film, lozenge, troche, tablet, cream, gel, ointment, liquid solution or suspension, powder, or capsule. In some embodiments, the unit dosage form can be a film or a tablet. In some embodiments, the tablet is an orally disintegrating tablet. In some embodiments, the pharmaceutical composition is formulated for transdermal, intradermal, intratracheal, intranasal, sublingual, or buccal administration. In some embodiments, the pharmaceutical composition is formulated for sublingual or buccal administration.

In some embodiments,
a pharmaceutical composition comprising an acid addition salt of apomorphine is administered to a subject.

In some embodiments,
a pharmaceutical composition of the present disclosure is administered to a subject in order to treat Parkinson's disease.

In some embodiments,
a pharmaceutical composition of the present disclosure is administered to a subject in order to treat restless leg syndrome.

In some embodiments,
a pharmaceutical composition of the present disclosure is administered to a subject in order to treat sexual dysfunction.

Other features and advantages of the invention will be apparent from the Drawings, Detailed Description, and the Claims.

### BRIEF DESCRIPTION OF THE DRAWINGS

FIG. 1 is a graph showing the X-ray powder diffraction (XRPD) patterns for apomorphine hydrochloride before compression (lower curve) and after compression (upper curve).
FIG. 2A is a graph showing the XRPD patterns for apomorphine glycolate.
FIG. 2B is a graph showing the XRPD patterns for apomorphine glycolate before compression (lower curve) and after compression (upper curve).
FIG. 3 is a graph showing differential scanning calorimetry (DSC) thermogram for apomorphine glycolate.
FIG. 4 is a graph showing thermogravimetric analysis (TGA) for apomorphine glycolate.
FIG. 5A is an image of the hot stage microscopy analysis of apomorphine glycolate at 169.0 °C.
FIG. 5B is an image of the hot stage microscopy analysis of apomorphine glycolate at 171.5 °C (melt onset).
FIG. 5C is an image of the hot stage microscopy analysis of apomorphine glycolate at 177.2 °C (discoloration).
FIG. 6 is a curve showing dynamic vapor sorption/desorption (DVS) for apomorphine glycolate.
FIG. 7 is a graph showing the XRPD patterns for apomorphine glycolate post DVS analysis.
FIG. 8A is a graph showing the XRPD patterns for apomorphine isobutyrate.
FIG. 8B is a graph showing the XRPD patterns for apomorphine isobutyrate before compression (lower curve) and after compression (upper curve).
FIG. 9 is a graph showing differential scanning calorimetry (DSC) thermogram for apomorphine isobutyrate.
FIG. 10 is a graph showing thermogravimetric analysis (TGA) for apomorphine isobutyrate.
FIG. 11A is an image of the hot stage microscopy analysis of apomorphine sulfamate at 123.4 °C.
FIG. 11B is an image of the hot stage microscopy analysis of apomorphine sulfamate at 128.0 °C (melt onset).
FIG. 11C is an image of the hot stage microscopy analysis of apomorphine sulfamate at 143.5 °C (melt complete).
FIG. 12 is a graph showing dynamic vapor sorption/desorption (DVS) analysis for apomorphine isobutyrate.
FIG. 13 is a graph showing the XRPD patterns for apomorphine isobutyrate post DVS analysis.
FIG. 14A is a graph showing the XRPD patterns for apomorphine sulfamate.
FIG. 14B is a graph showing the XRPD patterns for apomorphine sulfamate before compression (lower curve) and after compression (upper curve).
FIG. 15 is a graph showing differential scanning calorimetry (DSC) thermogram for apomorphine sulfamate.
FIG. 16 is a graph showing thermogravimetric analysis (TGA) for apomorphine sulfamate.
FIG. 17A is an image of the hot stage microscopy analysis of apomorphine sulfamate at 22.9 °C.
FIG. 17B is an image of the hot stage microscopy analysis of apomorphine sulfamate at 191.9 °C (melt onset).
FIG. 17C is an image of the hot stage microscopy analysis of apomorphine sulfamate at 194.6 °C (melt complete).
FIG. 18 is a graph showing a curve for dynamic vapor sorption/desorption (DVS) of apomorphine sulfamate.
FIG. 19 is a graph showing the XRPD patterns for apomorphine sulfamate post DVS analysis.
FIG. 20 is a graph showing the XRPD patterns for apomorphine methylbenzoate.
FIG. 21 is a graph showing the XRPD patterns for apomorphine napadisylate.
FIG. 22 is a graph showing the XRPD patterns for apomorphine glutarate.
FIG. 23 is a graph showing the XRPD patterns for apomorphine gentisate.
FIG. 24 is a graph showing the XRPD patterns for apomorphine cinnamate.
FIG. 25 is a graph showing the XRPD patterns for apomorphine 4-aminosalicylate.
FIG. 26 is a graph showing the XRPD patterns for apomorphine 4-acetamidobenzoate.
FIG. 27 is a graph showing the XRPD patterns for apomorphine 2,2-dichloroacetate.

### DETAILED DESCRIPTION

The present disclosure is made with the understanding that the description is to be considered as an exemplification of the claimed subject matter, and is not intended to limit the appended claims to the illustrated embodiments. The headings used throughout this disclosure are provided for convenience and are not to be construed to limit the claims in any way. Embodiments illustrated under any heading may be combined with embodiments illustrated under the same or any other heading where doing so would be within the scope of the claims.

Unless defined otherwise, all technical and scientific terms used herein have the same meaning as commonly understood by one of ordinary skill in the art.

As used herein, the term "comprising," or grammatical variants thereof, are to be taken as specifying the stated features, integers, steps or components but do not preclude the addition of one or more additional features, integers, steps, components or groups thereof. This term encompasses the terms "consisting of" and "consisting essentially of". The phrase "consisting essentially of," or grammatical variants thereof, when used herein are to be taken as specifying the stated features, integers, steps or components but do not preclude the addition of one or more additional features, integers, steps, components or groups thereof, but only if the additional features, integers, steps, components or groups thereof do not materially alter the basic and novel characteristics of the claimed composition or method.

As used herein, the singular forms "a", "an" and "the" are intended to include the plural forms as well, unless the context clearly indicates otherwise. It will be further understood that the terms "comprise" (and any form of comprise, such as "comprises" and "comprising"), "have" (and any form of have, such as "has" and "having"), "include" (and any form of include, such as "includes" and "including"), and "contain" (and any form contain, such as "contains" and "containing") are open-ended linking verbs. As a result, a method that "comprises", "has", "includes" or "contains" one or more steps or elements possesses those one or more steps or elements, but is not limited to possessing only those one or more steps or elements.

As used herein, the term "acid addition salt" in reference to an apomorphine salt (e.g., "an acid addition salt of apomorphine"), refers to a pharmaceutically acceptable salt of apomorphine, in which the apomorphine moiety is protonated and is positively charged, and the counterion is a Brønsted-Lowry conjugate base of an organic acid (e.g., isobutyric, 2,2-dichloroacetic, 4-acetamidobenzoic, 4-aminosalicylic, cinnamic, cyclamic, ethane disulfonic, gentisic, glutaric, methylbenzoic, 1,5-naphthalene disulfonic, acetic, lactic, pamoic, maleic, citric, malic, ascorbic, succinic, benzoic, palmitic, suberic, salicylic, tartaric, methanesulfonic, toluenesulfonic, or trifluoroacetic acid), a polymeric acid (e.g., as tannic acid, carboxymethyl cellulose, or alginic acid), or inorganic acid (e.g., sulfamic acid, hydrochloric acid, hydrobromic acid, sulfuric acid, or phosphoric acid). According to the invention, the acid is sulfamic acid. Other acids are for reference purposes only.

As used herein, the term "alkali salt" refers to a salt having lithium, sodium, or potassium as a counterion.

As used herein, the term "ammonium salt" refers to a salt having NH₄⁺ as a counterion.

As used herein, the term "bisulfite salt" refers to an alkali salt or ammonium salt of sulfurous acid (H₂SO₃). Non-limiting examples bisulfite salts include sodium bisulfite and potassium bisulfite.

As used herein, the term "Cmax" refers to an average observed maximum plasma concentration produced in a group of subjects (e.g., 10 or more) receiving an acid addition salt of apomorphine in an amount sufficient to produce an "on" state, where the amount of the film administered for each individual subject is the effective amount administered during up-titration of the individual subject (i.e., the Cmax accounting for variations in bioavailability) for a given route of administration (e.g., to oral mucosa, such as sublingual mucosa). The Cmax produced by the acid addition salt of apomorphine can be at least 2.6 ng/mL (e.g., 2.6 ng/mL to 20 ng/mL, 2.6 ng/mL to 15 ng/mL, or 2.6 ng/mL to 10 ng/mL).

As used herein, the term "effective amount" in reference to acid addition salt of apomorphine refers to a quantity of acid addition salt of apomorphine administered to a subject at once so as to produce rapid onset of action.

As used herein, the term "pharmaceutically acceptable" refers to compounds, salts, compositions, dosage forms and other materials which are useful in preparing a pharmaceutical composition suitable for veterinary or human pharmaceutical use.

As used herein, the term "rapid onset of action" refers to: (1) the circulating plasma concentration of at least 2.6 ng/mL of apomorphine in the subject within 45 minutes (e.g., within 30 minutes, within 25 minutes, within 20 minutes, within 19 minutes, within 18 minutes, within 17 minutes, within 16 minutes, or within 15 minutes ) after the acid addition salt of apomorphine film contacts oral mucosa (e.g., sublingual mucosa); or (2) the subject being in an "on" state within 45 minutes (e.g., within 30 minutes or within 20 minutes) after the acid addition salt of apomorphine film contacts oral mucosa (e.g., sublingual mucosa).

As used herein, the term "subject," to which administration is contemplated includes, but is not limited to, humans (i.e., any appropriate male or female). The "subject" may have independently been diagnosed with a disease or condition (e.g., Parkinson's disease) as defined herein, may currently be experiencing symptoms associated with a disease or condition (e.g., Parkinson's disease) or may have experienced symptoms in the past, may be at risk of developing a disease or condition (e.g., Parkinson's disease), or may be reporting one or more of the symptoms of a disorder, even though a diagnosis may not have been made. In some embodiments, the subject is a human. In some embodiments, the subject is a human having a dopamine-mediated disease or condition (e.g., Parkinson's disease). The subject may be diagnosed as having a dopamine-mediated disease or condition (e.g., Parkinson's disease) through the use of techniques known in the art, e.g., for Parkinson's disease, a unified Parkinson's disease rating scale (UPDRS, e.g., Movement Disorder Society-Sponsored Revision of UPDRS (MDS-UPDRS); see Goetz et al., Mov. Disord., 23:2129-2170, 2008) or Hoehn and Yahr scale may be used.

As used herein, the term "therapeutically effective amount" refers to an amount that is effective to elicit the desired biological or medical response, including the amount of a compound that, when administered to a subject for treating a disease or condition (e.g., Parkinson's disease), is sufficient to effect such treatment of the disease or condition (e.g., Parkinson's disease). The therapeutically effective amount may vary depending on the compound, the disease or condition (e.g., Parkinson's disease), and its severity, and the age, weight, etc. of the subject to be treated. The therapeutically effective amount may be in one or more doses (for example, a single dose or multiple doses may be required to achieve the desired treatment endpoint). A therapeutically effective amount may be considered to be given in a therapeutically effective amount if, in conjunction with one or more other agents, a desirable or beneficial result may be or is achieved. Suitable doses of any co-administered compounds may optionally be lowered due to the combined action, additive or synergistic, of the compound.

As used herein, the term "Tmax" refers to an average observed time to the maximum plasma concentration produced in a group of subjects (e.g., 10 or more) receiving an acid addition salt of apomorphine film in an amount sufficient to produce an "on" state, where the amount of the film administered for each individual subject is the lowest effective amount administered during up-titration of the individual subject (i.e., the Tmax accounting for variations in bioavailability) for a given route of administration (e.g., to oral mucosa, such as sublingual). Tmax for the films can be in the range from 20 minutes to 60 minutes (e.g., from 20 minutes to 50 minutes, from 20 minutes to 40 minutes, from 25 minutes to 60 minutes, from 25 minutes to 50 minutes, or from 25 minutes to 40 minutes).

The term "treating" (or derivatives thereof, such as "treatment"), as used herein in reference to a dopamine-mediated disease or condition (e.g., Parkinson's disease or a related symptom (e.g., an "off" state)) in a subject, is intended to refer to obtaining beneficial or desired results, e.g., clinical results, in a subject by administering a film to the subject. Beneficial or desired results may include alleviation or amelioration of one or more symptoms of a dopamine-mediated disease or condition, e.g., Parkinson's disease (e.g., switching a subject "on" from an "off" state, as assessed, e.g., in accordance with MDS-UPDRS); prevention of the occurrence of one or more symptoms of a dopamine-mediated disease or condition, e.g., Parkinson's disease (e.g., prevention of an "off" state). In some embodiments, "treating" includes one or more of the following: (a) inhibiting the disease or condition (e.g., Parkinson's disease) (for example, decreasing one or more symptoms resulting from the disease or condition, and/or diminishing the extent of the disease or condition); (b) slowing or arresting the development of one or more symptoms associated with the disease or condition (e.g., Parkinson's disease) (for example, stabilizing the disease or condition and/or delaying the worsening or progression of the disease or condition); and/or (c) relieving the disease or condition (e.g., Parkinson's disease) (for example, causing the regression of clinical symptoms, ameliorating the disease or condition, delaying the progression of the disease or condition, and/or increasing quality of life.)
**According to the invention, the acid is sulfamic acid. Other acids are for reference purposes only.**

In general, disclosed are acid addition salts of apomorphine. The acid addition salts of apomorphine (e.g., apomorphine sulfamate, apomorphine glycolate, or apomorphine isobutyrate) may be used as an active agent in pharmaceutical compositions (e.g., unit dosage forms). Advantageously, apomorphine sulfamate and apomorphine glycolate exhibit superior intrinsic dissolution rates in pH 4.5 acetate buffer and in artificial saliva at physiologically relevant 37 °C relative to the intrinsic dissolution rates of apomorphine hydrochloride under the same dissolution conditions.

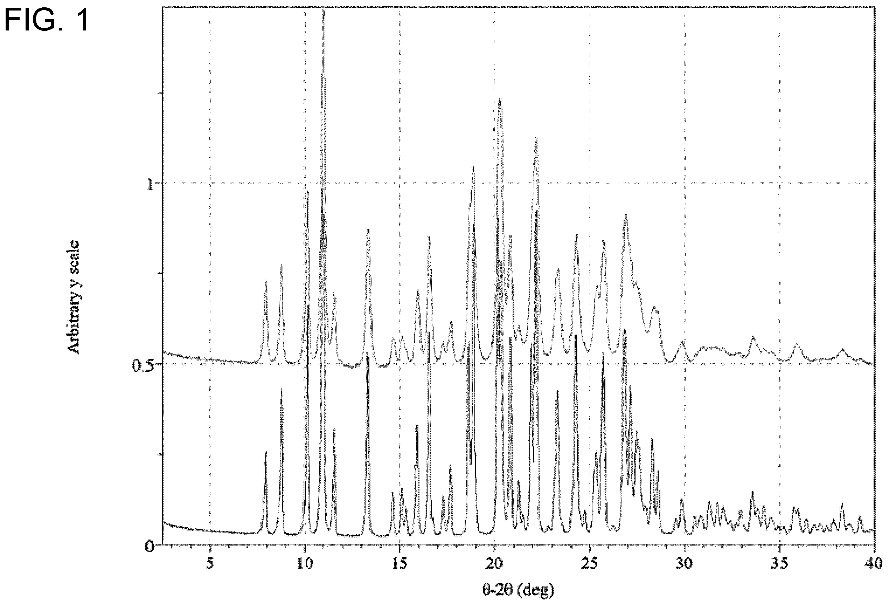

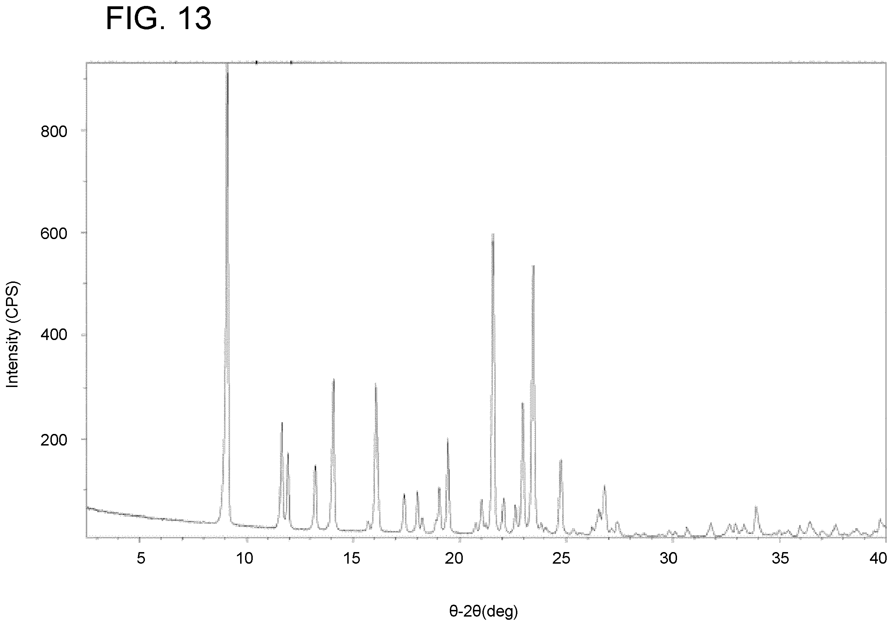

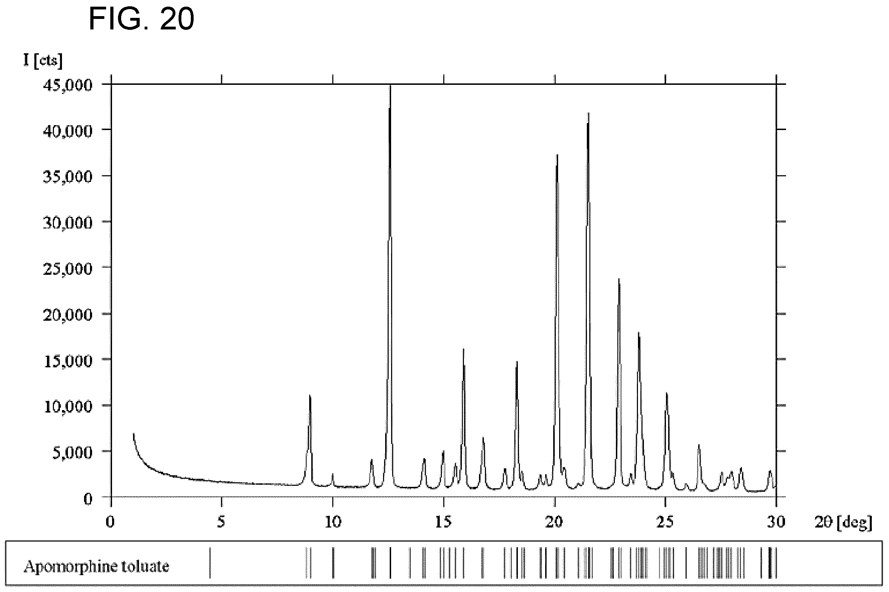

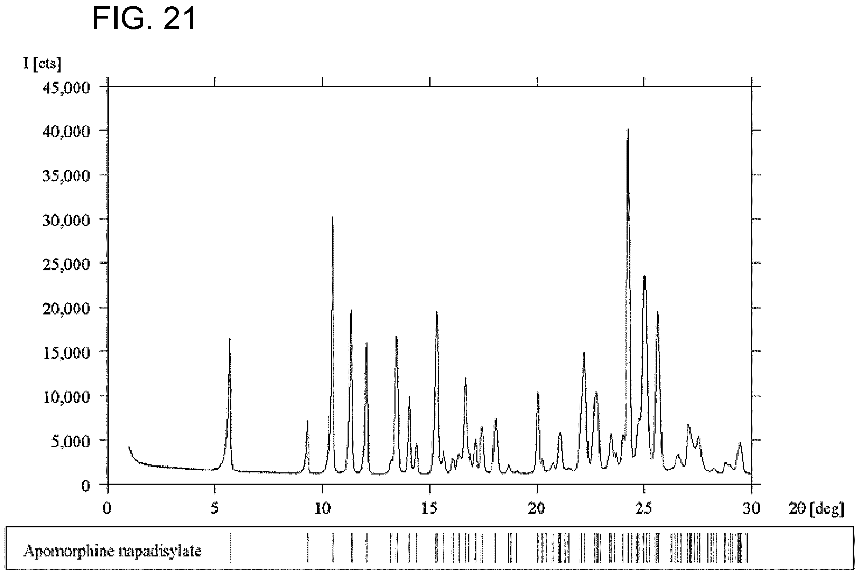

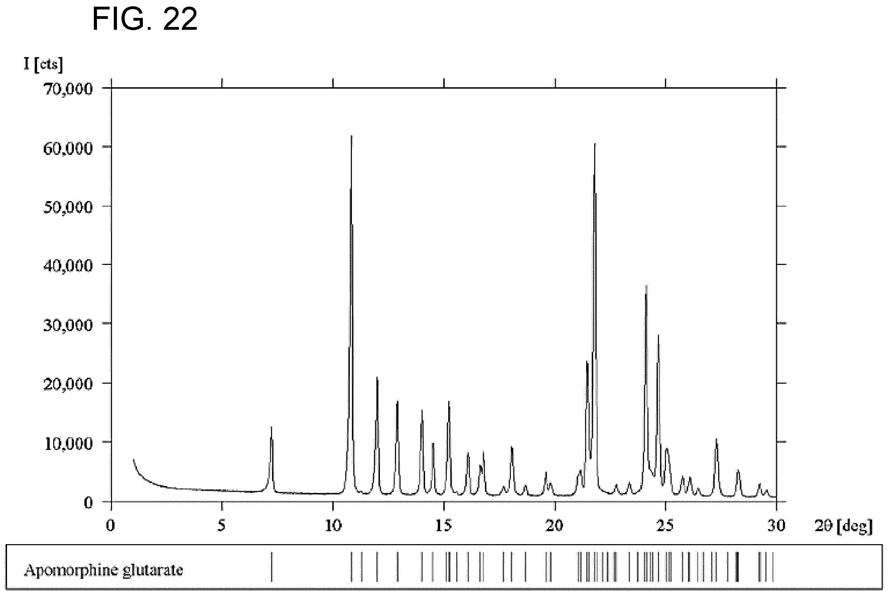

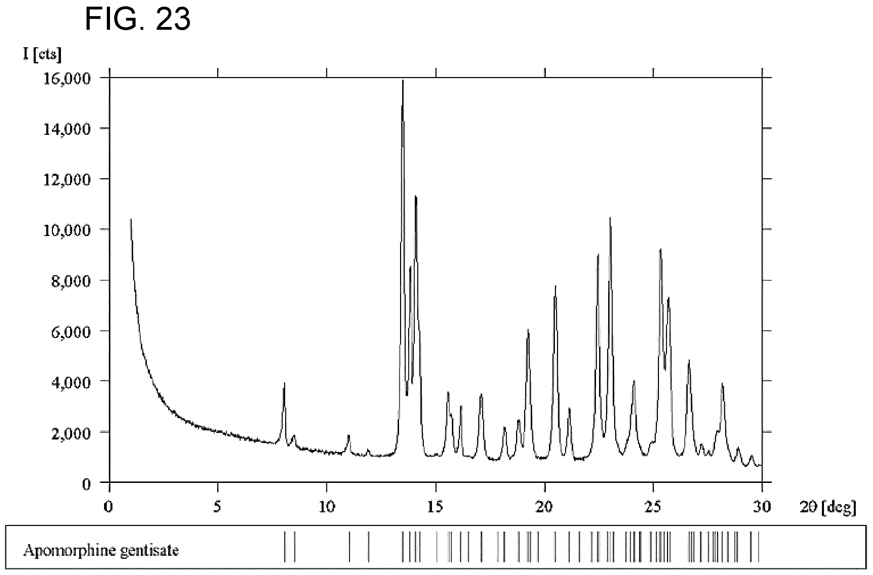

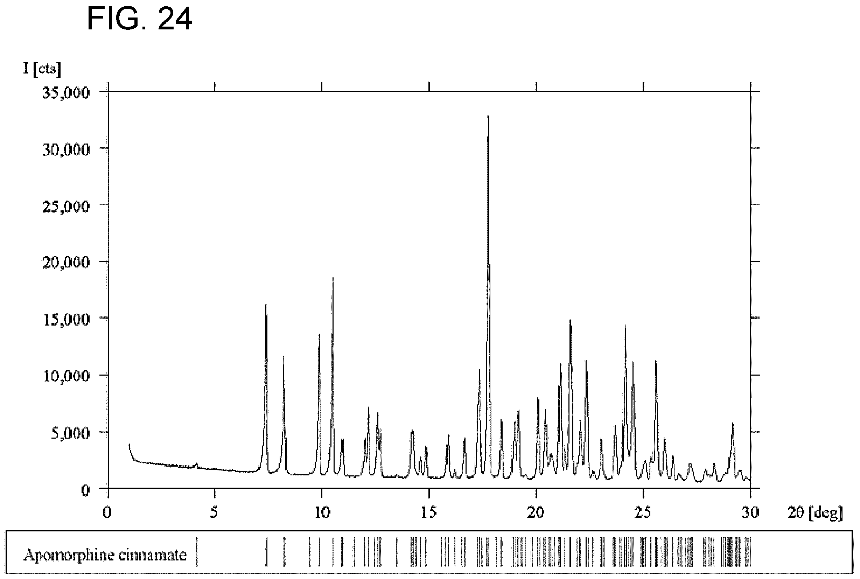

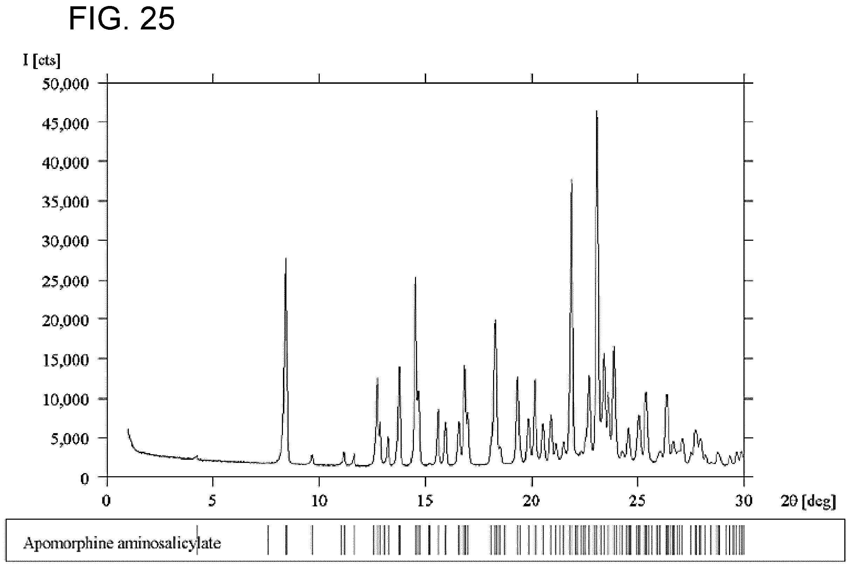

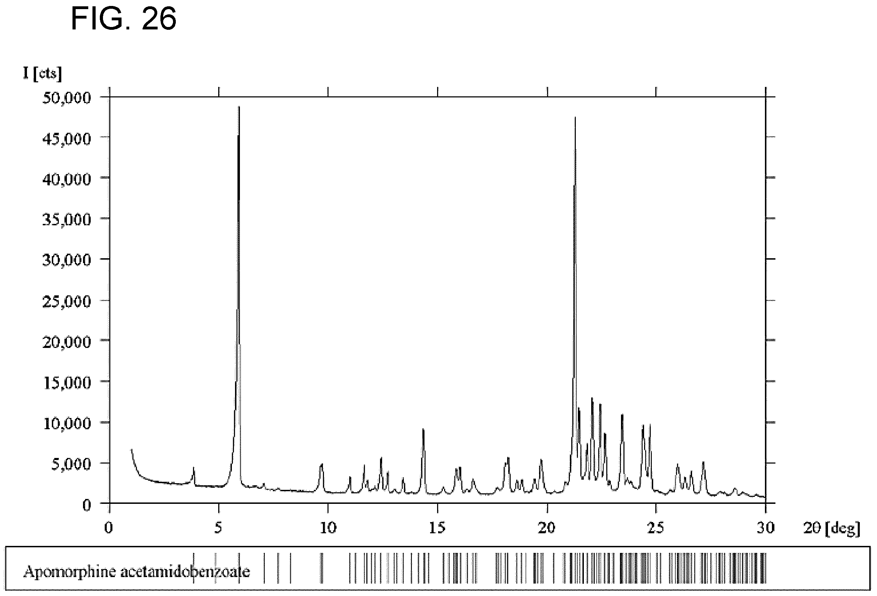

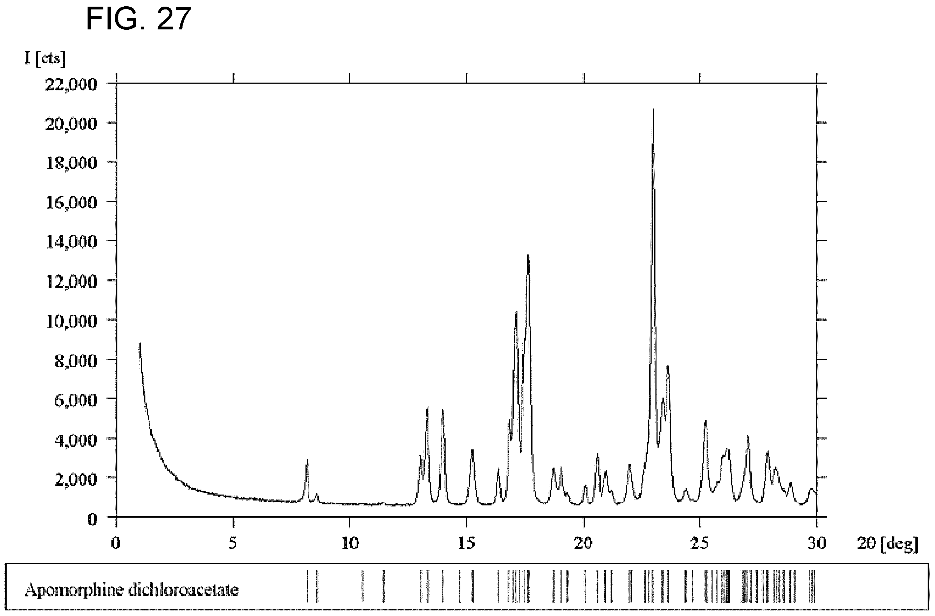

It has been surprisingly found that sulfamic acid, glycolic acid, isobutyric acid, 2,2-dichloroacetic acid, 4-acetamidobenzoic acid, 4-aminosalicylic acid, cinnamic acid, gentisic acid, glutaric acid, and methylbenzoic acid produce crystalline acid addition salts or apomorphine, whereas, upon reaction with free base apomorphine, caprylic acid, galacturonic acid, glucoheptonoic acid, hippuric acid, α-ketoglutaric acid, mandelic acid, mucic acid, palmitic acid, pamoic acid, pivalic acid, and sebacic acid produce oily and/or amorphous materials or unreacted acid guest complexes. X-ray powder diffraction patterns for certain acid addition salts of apomorphine are shown in FIG. 1, FIG. 2A, FIG. 2B,FIG. 7, FIG. 8A, FIG.8B, FIG. 13, FIG.14A, FIG. 14B, FIG. 19, FIG. 20, FIG. 21, FIG. 22, FIG. 23, FIG. 24, FIG. 25, FIG. 26, and FIG. 27.

The XRPD peaks for the crystalline form of apomorphine glycolate are found in Table 1 below.

**Table 1**

| **Diffraction Angle 2θ (°)** | **Relative intensity** |
|---|---|
| 10.3 | 97.5 |
| 12.4 | 73.7 |
| 16.1 | 46.1 |
| 16.4 | 24.7 |
| 20.6 | 21.0 |
| 21.3 | 100.0 |
| 21.7 | 78.3 |
| 23.3 | 26.2 |
| 23.7 | 98.9 |
| 24.7 | 20.8 |

The XRPD peaks for the crystalline form of apomorphine isobutyrate are found in Table 2 below.

**Table 2**

| **Diffraction Angle 2θ (°)** | **Relative intensity** |
|---|---|
| 9.1 | 100.0 |
| 11.6 | 25.0 |
| 14.1 | 34.0 |
| 16.1 | 35.5 |
| 19.4 | 22.1 |
| 21.6 | 75.9 |
| 23.0 | 30.9 |
| 23.4 | 65.4 |

The XRPD peaks for the crystalline form of apomorphine sulfamate are found in Table 3 below.

**Table 3**

| **Diffraction Angle 2θ (°)** | **Relative intensity** |
|---|---|
| 27.0 | 45.4 |
| 25.4 | 32.9 |
| 23.9 | 44.5 |
| 23.6 | 31.1 |
| 22.4 | 21.9 |
| 22.1 | 100 |
| 21.7 | 30.6 |
| 20.0 | 22.1 |
| 18.9 | 57.1 |
| 18.6 | 24 |
| 16.5 | 93 |
| 14.8 | 20.3 |
| 14.2 | 29.9 |
| 11.7 | 35.3 |
| 9.9 | 35.3 |

In some embodiments, the solid crystalline form of apomorphine glycolate is characterized by the X-ray powder diffraction (XRPD) pattern comprising one or more selected from those listed in Table 1. In some embodiments, the solid crystalline form of apomorphine isobutyrate is characterized by the X-ray powder diffraction (XRPD) pattern comprising one or more selected from those listed in Table 2. In some embodiments, the solid crystalline form of apomorphine sulfamate is characterized by the X-ray powder diffraction (XRPD) pattern comprising one or more selected from those listed in Table 3.

Those skilled in the art recognize that the measurements of the XRPD peak locations and/or intensity for a given crystalline form of the same compound will vary within a margin of error. The values of the diffraction angle 2θ allow appropriate error margins. For example, the diffraction angle 2θ of "10.0±0.3" denotes a range from 10.0+0.3 (i.e., 10.3) to 10.0-0.3 (i.e., 9.7). Depending on the sample preparation techniques, the calibration techniques applied to the instruments, human operational variation, and etc., those skilled in the art recognize that the appropriate error of margins for a XRPD can be ±0.5; ±0.4; ±0.3; ±0.2; ±0.1; ±0.05; or less. In some embodiments of the invention, the XRPD margin of error is ±0.2.

Unlike amorphous acid addition salts of apomorphine, acid addition salts of apomorphine disclosed herein and their solid crystalline forms may be suitable for formulating into a pharmaceutical composition. In some embodiments, acid addition salts of apomorphine disclosed herein and their solid crystalline forms may have superior stability to compression, for example, as measured by X-ray powder diffraction patterns. In addition, solid crystalline forms of an acid addition salt of apomorphine may have enhanced aqueous rate of dissolution relative to apomorphine hydrochloride.

In some embodiments, a solid crystalline form of an acid addition salt of apomorphine disclosed herein is a substantially pure crystalline form. A substantially pure crystalline compound is predominantly made up of a single crystalline phase. In some embodiments, over about 95% by weight of an acid addition salt of apomorphine is in a single crystalline phase. In some embodiments, less than about 2% by weight of an acid addition salt of apomorphine is present in an amorphous form. In some embodiments, less than about 1% of an acid addition salt of apomorphine is present in an amorphous form.

General methods for analyzing crystalline forms include crystal analysis by single crystal X-ray diffraction (SCXRD), X-ray powder diffraction (XRPD), differential scanning calorimetry (DSC), and/or thermogravimetric analysis (TGA).

Additional details of how to synthesize and use of the acid addition salts of apomorphine disclosed herein are provided below and in the Examples.

### Methods of Treatment

Representative examples of diseases and conditions treatable using acid addition salts of apomorphine disclosed herein include, but are not limited to, Parkinson's disease, restless leg syndrome, or sexual dysfunction.

The acid addition salts can be used to treat Parkinson's disease in a subject (e.g., treating hypomobility or an "off" episode" in a subject having Parkinson's disease). The treatment involves administering to the subject an acid addition salt of apomorphine disclosed herein, a solid crystalline form thereof, or a pharmaceutical composition disclosed herein. In some embodiments, an acid addition salt of apomorphine disclosed herein, a solid crystalline form thereof, or a pharmaceutical composition disclosed herein is administered to the oral mucosa of a subject (e.g., sublingual administration or buccal administration). In some embodiments, an acid addition salt of apomorphine disclosed herein, a solid crystalline form thereof, or a pharmaceutical composition disclosed herein is administered in a therapeutically effective amount, for example, to produce at least the minimum effective plasma concentration of apomorphine (i.e., at least 2.6 ng/ml). The pharmaceutical compositions described herein can be used in the methods described herein.

In some embodiments, the treatment of Parkinson's disease comprises treating acute or intermittent "OFF" episodes associated with Parkinson's disease. In some embodiments, the acute or intermittent "OFF" episodes associated with Parkinson's disease comprises at least one of end-of-dose wearing "OFF" (including early morning "OFF"), partial "OFF," delayed "OFF," No-ON "OFF," or unpredictable "OFF."

### Pharmacokinetics/Pharmacodynamics

The minimum effective concentration of apomorphine can be achieved within 30 minutes of administering an acid addition salt of apomorphine disclosed herein to the subject. An acid addition salt of apomorphine disclosed herein may be administered in a pharmaceutical composition and/or as a solid crystalline form disclosed herein. In some embodiments, an apomorphine Cmax of less than 30 ng/ml (e.g., less than 20 ng/ml, less than 10 ng/ml, less than 7 ng/ml or less than 5 ng/ml) is produced after the administering step. In some embodiments, apomorphine Cmax may be in the range 2.6 ng/ml to 30 ng/ml (e.g., 2.6 ng/ml to 20 ng/ml, 2.6 ng/ml to 10 ng/ml, or 2.6 ng/ml to 5 ng/ml). In some embodiments, Tmax for observed for the methods of disclosed herein is in the range of 10 minutes to 1 hour (e.g., 20 minutes to 1 hour, or 20 minutes to 50 minutes). The identification of an appropriate dose for the subject may be performed using methods known in the art, e.g., titration. In some embodiments, titration is uptitration. Uptitration may involve administering to the subject a first predetermined dosage of apomorphine (e.g., 12.5 ± 2.5 mg of an acid addition salt of apomorphine; 20.0 ± 5.0 mg of an acid addition salt of apomorphine; or 30.0 ± 5.0 mg of an acid addition salt of apomorphine), and determining if a therapeutically effective amount of apomorphine was administered; if the amount of administered apomorphine was a therapeutically effective amount. The determination if an effective amount of apomorphine was administered in any one of the above uptitration steps can be executed in accordance with methods known in the art, e.g., by evaluating UPDRS (e.g., UPDRS Part III) for the subject within a predetermined period (e.g., 30 minutes or 45 minutes) after administering apomorphine or by measuring apomorphine plasma concentration in a blood sample obtained from the subject within a predetermined period (e.g., 30 minutes or 45 minutes) after administering apomorphine.

### Pharmaceutical Unit Dosage Forms

The acid addition salts of apomorphine described herein may be formulated into pharmaceutical compositions for administration to subjects. The present disclosure features a pharmaceutical composition comprising an acid addition salt of apomorphine in admixture with a suitable diluent, carrier, or excipient. Non-limiting examples of the unit dosage forms include a film, lozenge, orally disintegrating tablet, gel, liquid solution, suspension, or powder.

The acid addition salts of apomorphine described herein may be formulated for transdermal, intradermal, intratracheal, intranasal, sublingual, buccal, or inhalation administration.

To reduce the occurrence of adverse events related to systemic spikes of acid addition salt of apomorphine plasma levels (e.g., somnolescence, nausea, yawning, headache, or hyperhidrosis), pharmaceutical compositions disclosed herein (e.g., pharmaceutical unit dosage forms) may produce circulating levels that are sufficiently high to be therapeutically effective and are sufficiently low to reduce the occurrence of adverse events. For example, films may produce a Cmax in the range of 2.6 ng/mL to 20 ng/mL, 2.6 ng/mL to 15 ng/mL, or 2.6 ng/mL to 10 ng/mL upon administration to oral mucosa (e.g., sublingual mucosa).

In some embodiments, a pharmaceutical unit dosage form described herein contains from 2 mg to 60 mg of an acid addition salt of apomorphine (e.g., from 8 mg to 45 mg of an acid addition salt of apomorphine). Certain exemplary pharmaceutical unit dosage forms may contain 10.0 ± 2.0 mg, 12.5 ± 2.5 mg, 15.0 ± 2.5 mg, 17.5 ± 2.5 mg, 20.0 ± 5.0 mg, 25.0 ± 5.0 mg, 30.0 ± 10.0 mg, 30.0 ± 5.0 mg, 35.0 ± 10.0 mg, or 35.0 ± 5.0 mg of an acid addition salt of apomorphine. The pharmaceutical unit dosage form may contain from 10% (w/w) to 60% (w/w) of an acid addition salt of apomorphine (e.g., from 20% (w/w) to 60% (w/w), from 30% (w/w) to 60% (w/w), or from 40% (w/w) to 60% (w/w)) relative to the weight of the pharmaceutical unit dosage form.

The pharmaceutical unit dosage forms described herein can include apomorphine microparticles having a D50 of from 1 µm to 500 µm (e.g., from 1 µm to 100 µm or 1 µm to 50 µm). The starting microparticles can be microspheres can be made of an acid addition salt of apomorphine and predominantly crystalline. An acid addition salt of apomorphine can be encapsulated in the microsphere or included in a dissolved drug microsphere. In an alternative approach, the pharmaceutical formulations described herein can include apomorphine particles having an effective particle size of less than about 1 µm (i.e., nanoparticulate formulations). An acid addition salt of apomorphine can be encapsulated in the microsphere or included in a dissolved-drug microsphere. These apomorphine particles can be made by using any method known in the art for achieving the desired particle sizes. Useful methods include, for example, milling, homogenization, supercritical fluid fracture, or precipitation techniques. Exemplary methods are described in U.S. Patent Nos. 4,540,602; 5,145,684; 5,518,187; 5,718,388; 5,862,999; 5,665,331; 5,662,883; 5,560,932; 5,543,133; 5,534,270; and 5,510,118; 5,470,583, each of which is specifically incorporated by reference.

### Rapid Absorption Solid Dosage Forms

The pharmaceutical compositions described herein can provide a rapid-dissolving, rapid absorption solid dosage form that includes an acid addition salt of apomorphine. Non-limiting examples of a rapid absorption solid dosage form include, for example, orally disintegrating tablets, orally disintegrating films, and intranasal or inhalable dosage forms, such as powders. An acid addition salt of apomorphine may achieve fast absorption by virtue of having high aqueous dissolution rate. In some embodiments, the rapid absorption solid further comprises a pH neutralizing agent. Inclusion of a pH neutralizing agent may facilitate absorption of apomorphine. Without wishing to be bound by theory, it is believed that transmucosal uptake of apomorphine is better for free-base apomorphine than for cationic apomorphine (e.g., as found in acid addition salts of apomorphine). Accordingly, a pharmaceutical composition disclosed herein may produce pH of about 5 to about 9, upon mixing with 1 mL of unbuffered water at about 25 °.

### Films

A pharmaceutical composition disclosed herein may be provided as a unit dosage form that is a film comprising an acid addition salt of apomorphine. An acid addition salt of apomorphine may be present in the films as a solid solution in a polymeric carrier. Without wishing to be bound by theory, a solid solution of an acid addition salt of apomorphine in a polymeric carrier provides acid addition salt of apomorphine in pre-dissolved form and thus can provide a more rapid onset of action, as the dissolution of the polymeric carrier is expected to control the release of acid addition salt of apomorphine in the oral media (e.g., artificial saliva as is described herein). Films may furthermore undergo rapid dissolution or rapid disintegration in the oral media (e.g., saliva), e.g., within about 10 minutes (preferably, within about 5 minutes; more preferably, within about 3 minutes; yet more preferably, within about 2 minutes; and still more preferably, within about 1 minute).

### Nasal Formulation

An acid addition salt described herein may also be formulated for nasal administration. For intranasal therapeutic administration, an acid addition salt of apomorphine described herein may be formulated as aerosols, drops, gels, and powders. The formulations may be provided in a single or multidose form. In the case of a dropper or pipette, dosing may be achieved by the subject administering an appropriate, predetermined volume of the solution or suspension. In the case of a spray, this may be achieve, e.g., by means of a metering atomizing spray pump. The acid addition salts of apomorphine described herein may also be formulated for aerosol administration. For aerosol administration, the acid addition salt of apomorphine will generally have a small particle size, e.g., on the order of five microns or less.

### Pharmaceutical Excipients

Provided herein are formulations comprising an acid addition salt of apomorphine, and one or more pharmaceutically acceptable excipients. Non-limiting examples of pharmaceutically acceptable excipients includes, any binder, filler, adjuvant, carrier, solubilizer, antioxidant, buffering agent, permeation enhancer, hydrolyzed starches, excipient, glidant, sweetening agent, diluent, preservative, dye/colorant, flavor enhancer, surfactant, wetting agent, dispersing agent, suspending agent, stabilizer, isotonic agent, solvent, emulsifier, anti-caking agent, flavor, desiccants, plasticizers, vehicle, disintegrants, or lubricant which has been approved by the United States Food and Drug Administration as being acceptable for use in humans or domestic animals.

The formulations described herein can include an antioxidant system (e.g., a combination of at least two antioxidants) in a unit dosage form including an acid addition salt described herein. Antioxidants are known in the art. Non-limiting examples of antioxidants that may be included in the dosage form are thiols (e.g., aurothioglucose, dihydrolipoic acid, propylthiouracil, thioredoxin, glutathione, cysteine, cystine, cystamine, thiodipropionic acid), sulphoximines (e.g., buthionine-sulphoximines, homo-cysteine-sulphoximine, buthionine-sulphones, or penta-, hexa- or heptathionine-sulphoximine), metal chelators (e.g, α-hydroxy-fatty acids, palmitic acid, phytic acid, lactoferrin, citric acid, lactic acid, succinic acid, malic acid, humic acid, bile acid, bile extracts, bilirubin, biliverdin, EDTA, EGTA, or DTPA or a salt thereof), metabisulfite salts (e.g., sodium metabisulfite or potassium metabisulfite), bisulfite salts (e.g., sodium bisulfite or potassium bisulfite), sodium thiosulfate, vitamins and vitamin derivatives (e.g., vitamin E, vitamin C, ascorbyl palmitate, Mg ascorbyl phosphate, and ascorbyl acetate), phenols (e.g., butylhydroxytoluene, butylhydroxyanisole, ubiquinol, nordihydroguaiaretic acid, or trihydroxybutyrophenone), benzoates (e.g., coniferyl benzoate), cyclodextrins (e.g., β-cyclodextrin or sulfobutyl-β-cyclodextrin), uric acid, mannose, propyl gallate, selenium (e.g., selenium-methionine), stilbenes (e.g., stilbene oxide and trans-stilbene oxide), and combinations thereof.

### EXAMPLES

The following examples are put forth to provide those of ordinary skill in the art with a description of how the compositions and methods described herein may be used, made, and evaluated, and are intended to be purely exemplary of the invention and are not intended to limit the scope of what the inventors regard as their invention.

### Example 1. Salt Preparation

Acid counterions summarized in Table 4, were chosen for preparing a salt with the free base, based on known p*K*ₐ values. Over 70 crystallization experiments were conducted. These experiments generally involved direct addition of approximately one half or one molar equivalent of the counterion to free base in solution. Acid addition salt of apomorphine was either prepared in bulk and sub-sampled for salt experiments or prepared in situ prior to acid addition (with the assumption of 100% yield for calculation of molar equivalents). Solid materials were harvested if precipitation of sufficient quantity occurred, or additional steps such as (but not limited to) cooling, anti-solvent addition, and/or slurrying were performed to induce crystallization or increase yields. The products were qualitatively evaluated for crystallinity by polarized light microscopy and/or XRPD. Select non-crystalline materials were exposed to additional conditions in an attempt to crystallize the salts. Attempts to generate crystalline caprylate, galacturonate, glucoheptonoate, hippurate, α-ketoglutarate, mandelate, mucate, palmitate, pamoate, pivalate, and sebacate salts were not successful; experiments with the respective acids resulted in oily/amorphous materials or unreacted acid guest. For suspected solvates, vacuum drying helped to identify unsolvated crystal forms of the potential salts. Solution ¹H NMR (nuclear magnetic resonance) spectroscopy was used to confirm composition/stoichiometry, verify that chemical degradation did not occur, and evaluate the amount of solvent present.

Crystalline, unsolvated salts with negligible impurities were selected for further evaluation. Other salts were not further evaluated based on undesired properties (e.g., the presence of solvent, significant impurities, difficulty to reproduce, or low crystallinity). Ideal solid forms would be crystalline materials that exhibit improved aqueous solubility (normalized to free base) as compared to the HCl salt (~21 mg/mL), maintain neutral pH upon dissolution, exhibit suitable physical stability at the elevated humidity conditions evaluated, and exhibit a melting temperature above approximately 125 ºC. The approximate aqueous solubility and pH of resulting solutions were tested; the physical stability of the materials upon exposure to 97% RH was investigated; and approximate melting points were assessed by heating. The results from the aqueous solubility and physical stability evaluation are summarized in Table 4 below.

**Table 4**

| **Aqueous Solubility and Physical Property Assessment of Apomorphine Salts** | | | |
|---|---|---|---|
| **Identifier** | **Approximate Aqueous Solubility (mg/mL)** | **Approximate pH of Aqueous Solution** | **Melting Point Onset (°C)** |
| 2,2-dichloroacetate | 3 | 5 | 160 |
| 4-acetamidobenzoate | 4 | 5 | 155 |
| Cinnamate | < 2 | - | 200 |
| Gentisate | 1 (ppt.) | 4.6 | 125 |
| Glutarate | 3 | 6 | 190 |
| Glycolate | > 133 | 5-6 | 172 |
| Isobutyrate | 6 | 6-7 | 128 |
| Methylbenzoate | < 1 | - | 170 |
| Sulfamate | > 179 | 5 | 192 |

| | | | |
|---|---|---|---|
| The sulfamate is according to the invention. The other salts are for reference. | | | |

The aqueous solubility of apomorphine glycolate and apomorphine sulfamate are significantly higher than the solubility observed for the other salts listed in Table 4. High aqueous solubility can be advantageous for increasing sublingual and buccal bioavailability of the apomorphine following sublingual or buccal administration.

### Example 2. Salt Preparation and Characterization

### X-ray Powder Diffraction (XRPD) - Transmission Geometry

XRPD patterns were collected with a PANalytical X'Pert PRO MPD diffractometer using an incident beam of Cu radiation produced using an Optix long, fine-focus source. An elliptically graded multilayer mirror was used to focus Cu Kα X-rays through the specimen and onto the detector. Prior to the analysis, a silicon specimen (NIST SRM 640e) was analyzed to verify the observed position of the Si 111 peak is consistent with the NIST-certified position. A specimen of the sample was sandwiched between 3-µm-thick films and analyzed in transmission geometry. A beam-stop, short antiscatter extension, and antiscatter knife edge were used to minimize the background generated by air. Soller slits for the incident and diffracted beams were used to minimize broadening from axial divergence. Diffraction patterns were collected using a scanning position-sensitive detector (X'Celerator) located 240 mm from the specimen and Data Collector software v. 2.2b.

### X-ray Powder Diffraction (XRPD) - Reflection Geometry (for samples in limited quantity)

XRPD patterns were collected with a PANalytical X'Pert PRO MPD diffractometer using an incident beam of Cu Kα radiation produced using a long, fine-focus source and a nickel filter. The diffractometer was configured using the symmetric Bragg-Brentano geometry. Prior to the analysis, a silicon specimen (NIST SRM 640e) was analyzed to verify the observed position of the Si 111 peak is consistent with the NIST-certified position. A specimen of the sample was prepared as a thin, circular layer centered on a silicon zero-background substrate. Antiscatter slits (SS) were used to minimize the background generated by air. Soller slits for the incident and diffracted beams were used to minimize broadening from axial divergence. Diffraction patterns were collected using a scanning position-sensitive detector (X'Celerator) located 240 mm from the sample and Data Collector software v. 2.2b.

### Differential Scanning Calorimetry (DSC)

DSC was performed using a TA Instruments Q2000 differential scanning calorimeter. Temperature calibration was performed using NIST-traceable indium metal. The sample was placed into an aluminum Tzero crimped pan (T0C), and the weight was accurately recorded. A weighed aluminum pan configured as the sample pan was placed on the reference side of the cell.

### Thermal Gravimetric Analysis (TGA)

TG analyses were performed using a TA Instruments Q5000 IR thermogravimetric analyzer. Temperature calibration was performed using nickel and Alumel^{™}. Each sample was placed in an aluminum pan. The sample was hermetically sealed, the lid pierced, then inserted into the TG furnace. The furnace was heated under nitrogen.

### Hot Stage Microscopy

Hot stage microscopy was performed using a Linkam hot stage (FTIR 600) mounted on a Leica DM LP microscope equipped with a SPOT Insight^{™} color digital camera. Temperature calibrations were performed using USP melting point standards. Samples were placed on a cover glass, and a second cover glass was placed on top of the sample. As the stage was heated, each sample was visually observed at a magnification of 20x utilizing a 0.40 NA objective with crossed polarizers and a first order red compensator. Images were captured using SPOT software (v. 4.5.9).

### Dynamic Vapor Sorption/Desorption (DVS)

Dynamic vapor sorption (DVS) data were collected on a VTI SGA-100 Vapor Sorption Analyzer. NaCl and PVP were used as calibration standards. Samples were not dried prior to analysis. Sorption and desorption data were collected over a range from 5% to 95% RH at 10% RH increments under a nitrogen purge. The equilibrium criterion used for analysis was less than 0.0100% weight change in 5 minutes with a maximum equilibration time of 3 hours. Data were not corrected for the initial moisture content of the samples.

### Single Crystal Data Collection (SCXRD)

Standard uncertainty in this report is written in crystallographic parenthesis notation, e.g. 0.123(4) is equivalent to 0.123 ± 0.004. Calculated XRPD patterns were generated for Cu radiation using MERCURY [7] and the atomic coordinates, space group, and unit cell parameters from the single crystal structure. The atomic displacement ellipsoid diagrams were prepared using MERCURY. Atoms are represented by 50% probability anisotropic thermal ellipsoids.

### Preparation of Acid Addition Salts of Apomorphine

### General Procedure

A vessel was charged with apomorphine free base dissolved in diethyl ether. To this was added a molar equivalent of acid in diethyl ether. The vessel was covered with aluminum foil to protect from light, and the mixture was left to slurry at ambient or low temperature until sufficient solids had precipitated. The solids were then collected by filtration and dried.

### Preparation of Glycolic Acid Addition Salt of Apomorphine (Reference)

A vessel was charged with 947.5 mg of apomorphine free base and slurried in 100 mL of diethyl ether. A molar equivalent of glycolic acid (268.9 mg) was dissolved in diethyl ether (19 mL) and added to the apomorphine free base slurry. Upon contact it was noted that white precipitates formed. The vessel was "seeded" with a small quantity of Glycolate Form A (7116-02-02). The vessel was covered with aluminum foil to protect from light. A stir bar was added and the sample was left to slurry for 3 days at room temperature. The solids were recovered by filtration and rinsed with 15 mL of diethyl ether. The material (7179-04-01) was vacuum dried, at ambient, for approximately 4.5 hours.

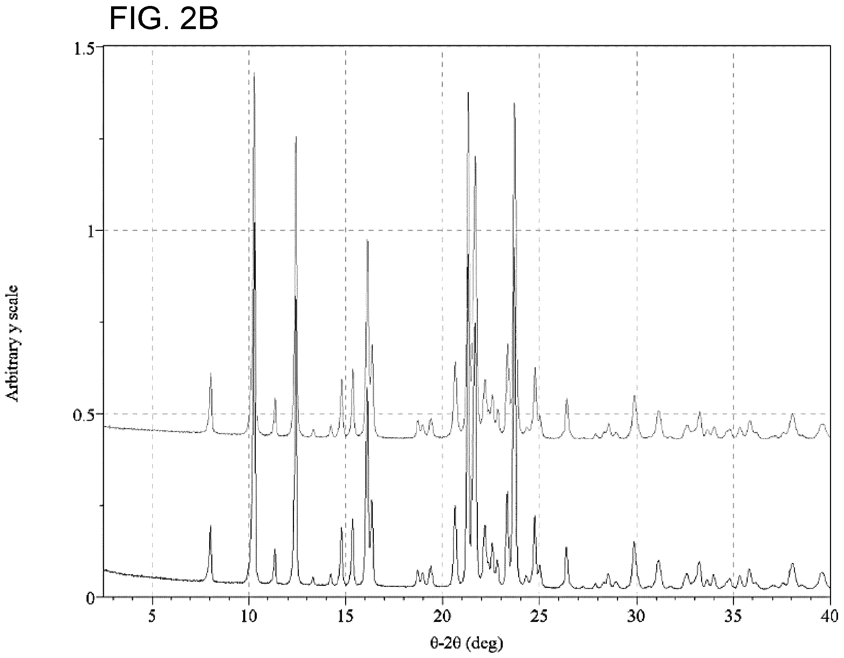

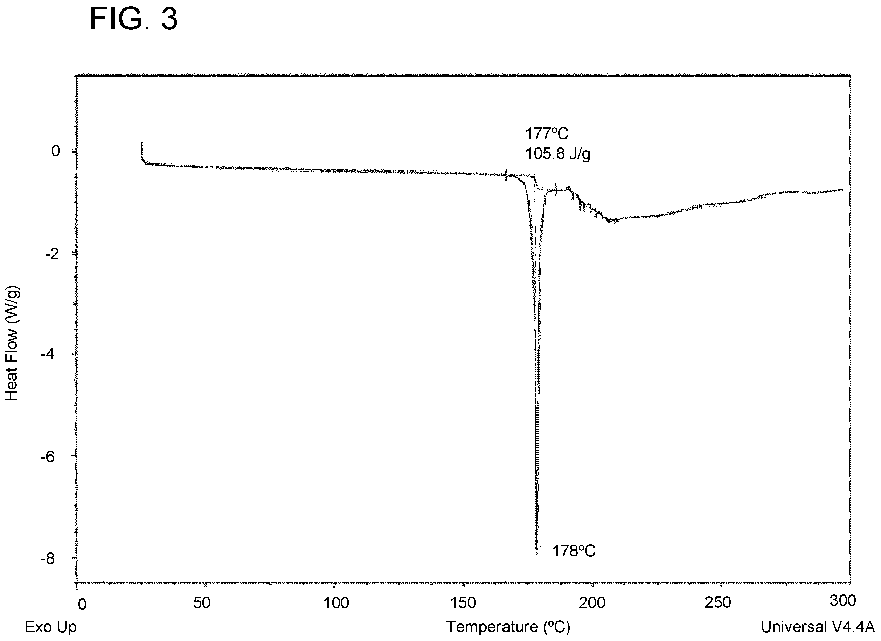

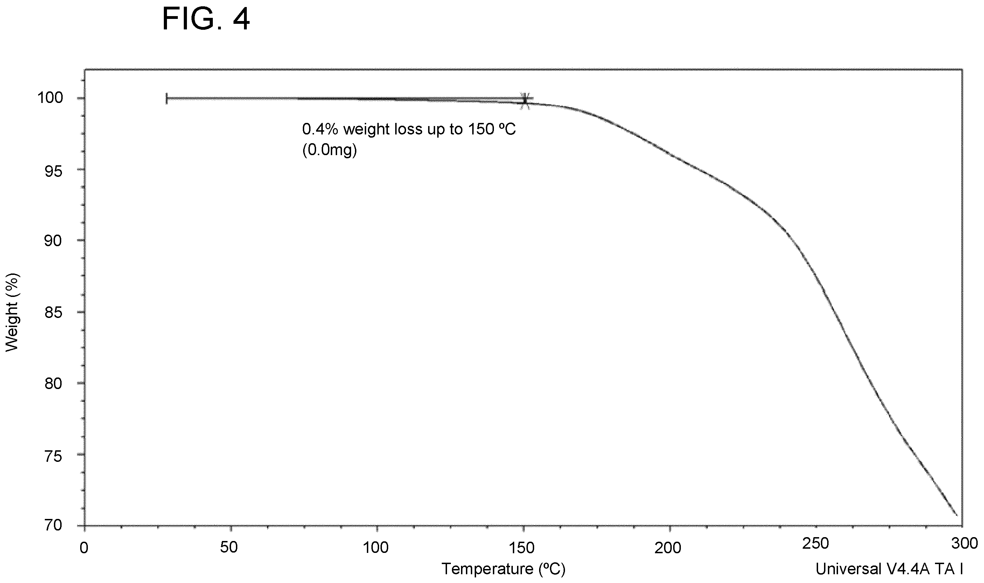

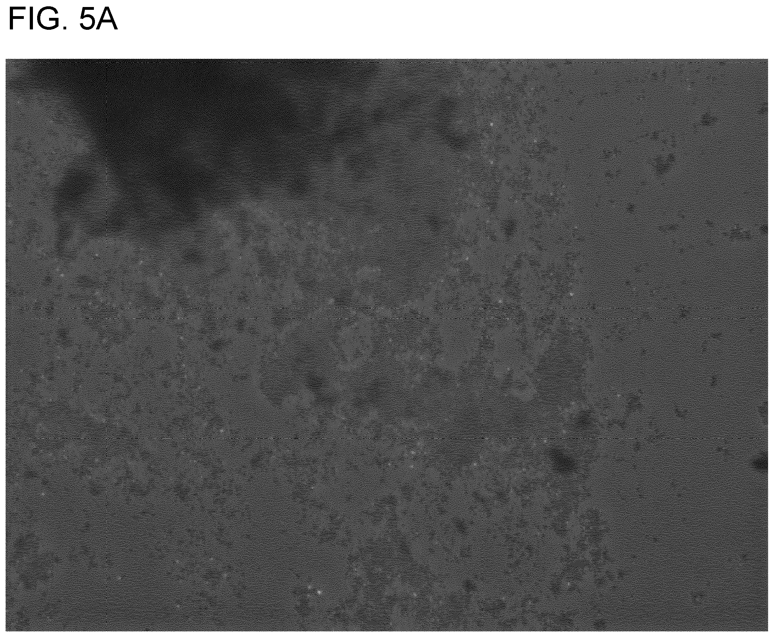

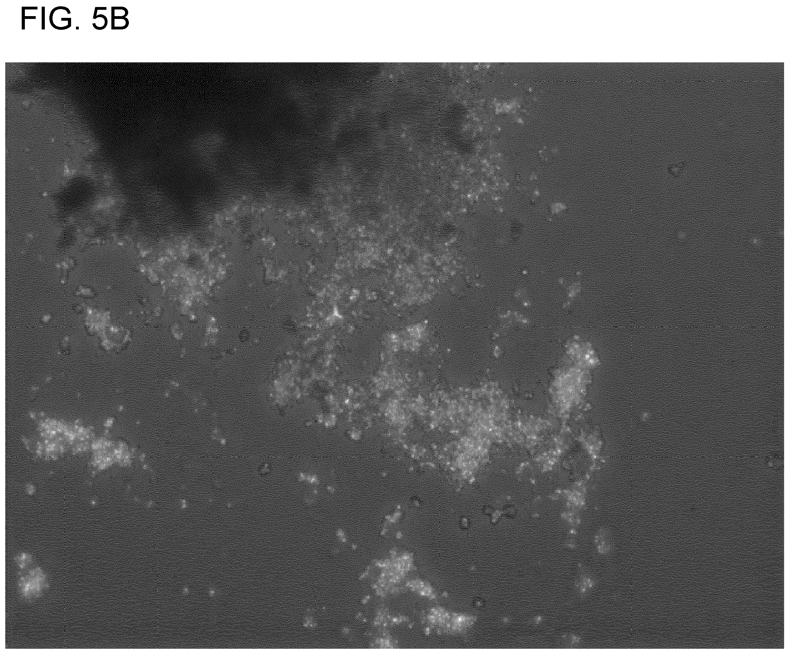

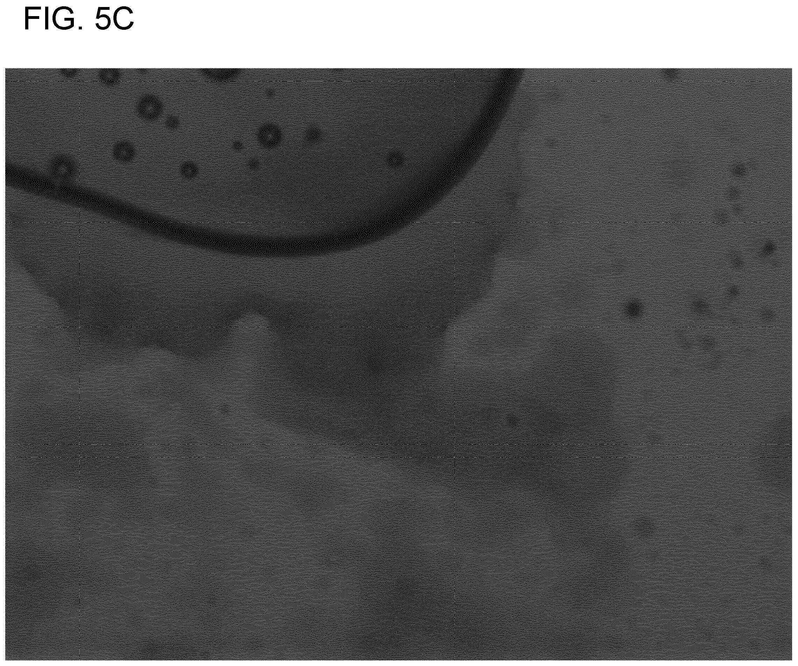

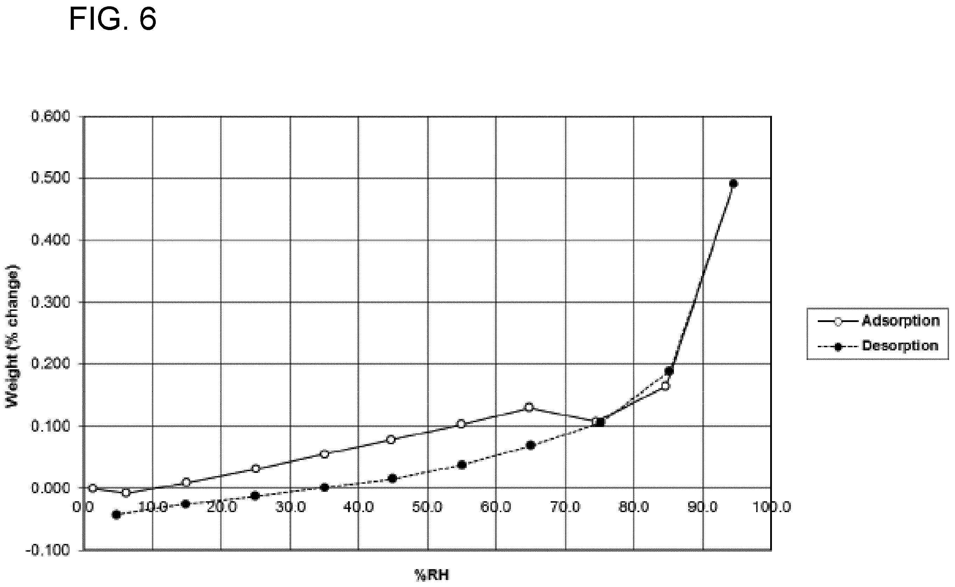

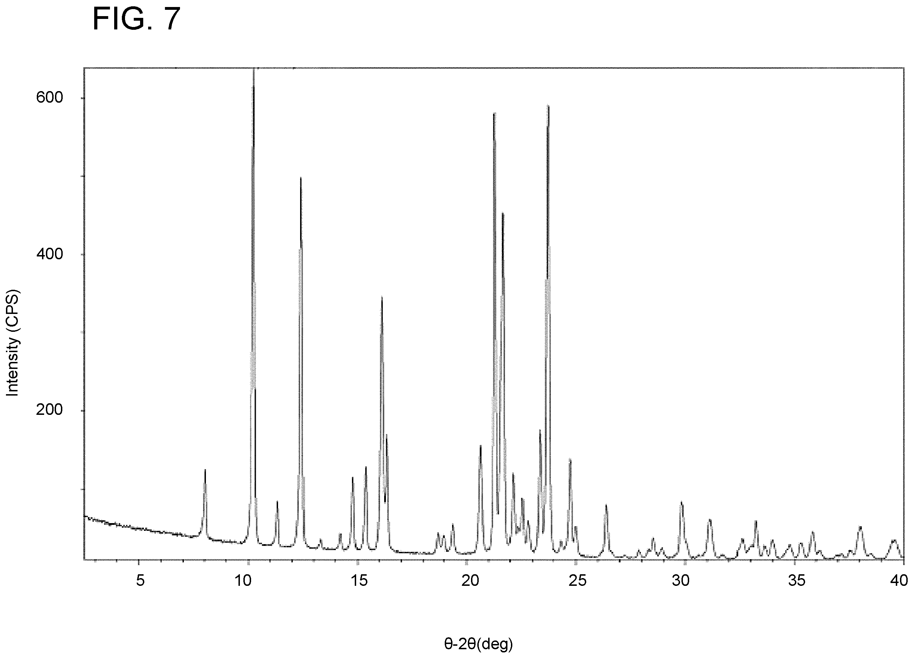

Sample 7179-04-01 exhibited a melting point of 172 °C and was analyzed by XRPD (FIG. 2), DSC (FIG. 3), TGA (FIG. 4), hot stage microscopy (FIG. 5), and DVS (FIG. 6). The DSC analysis revealed the presence of an endotherm at 177 °C, and TGA analysis revealed that there is a 0.4% weight loss up to 150 °C. Hot stage microscopy showed an onset of melting at 171.5 °C and discoloration at 177.2 °C. DVS analysis revealed 0.0% weight change at 5% equilibration; 0.5% weight gain from 5 to 95% RH; and 0.5% weight loss from 95 to 5% RH. The XRPD pattern post DVS analysis is shown in FIG. 7.

### Preparation of Sulfamic Acid Addition Salt of Apomorphine

A vessel was charged with 1.5172 g of apomorphine free base. A molar equivalent of sulfamic acid was dissolved in a 9:1 v/v ethanol/water solvent system at 50 °C and then added to the apomorphine solid resulting in a clear solution. The solution was contacted with 30 mL of diethyl ether and observed to be slightly turbid. The sample was "seeded" with Sulfamate Material A (7116-70-02). An increase in turbidity was observed after approximately 10 minutes. An additional 30 mL of diethyl ether was added, resulting in formation of tacky oil. The vessel was covered with aluminum foil to protect from light and slurried at ambient overnight. The solids were recovered by filtration and vacuum dried, at ambient, overnight.

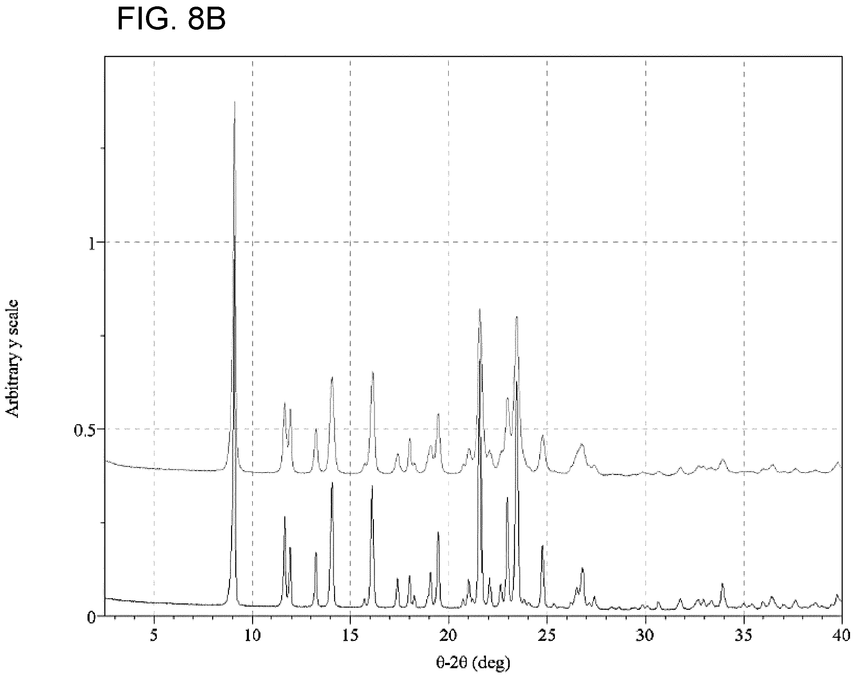

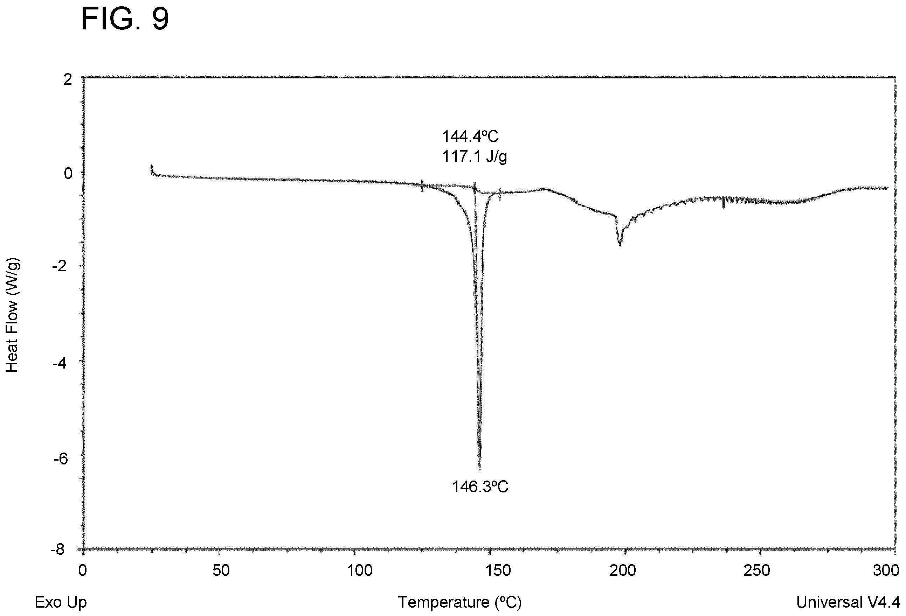

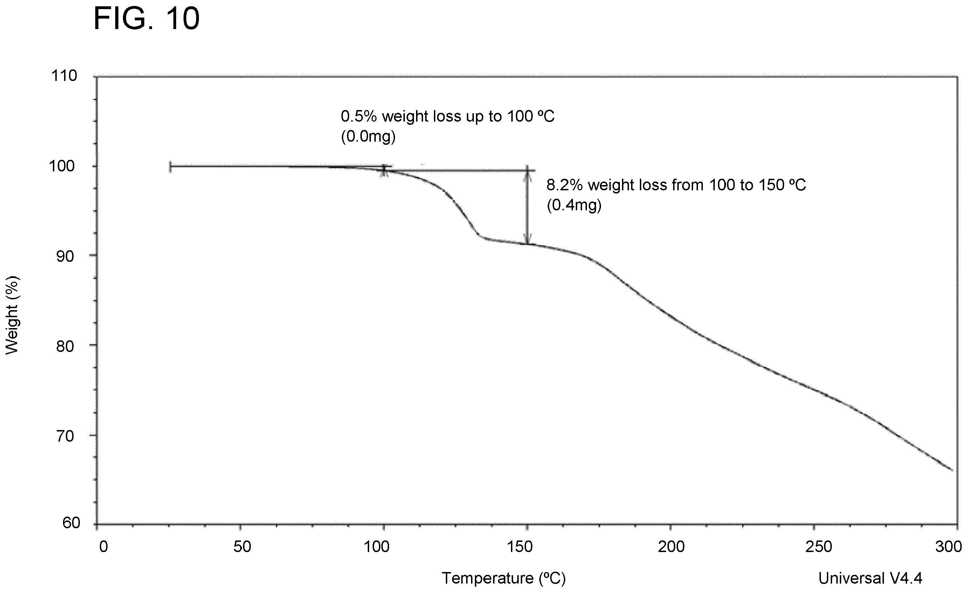

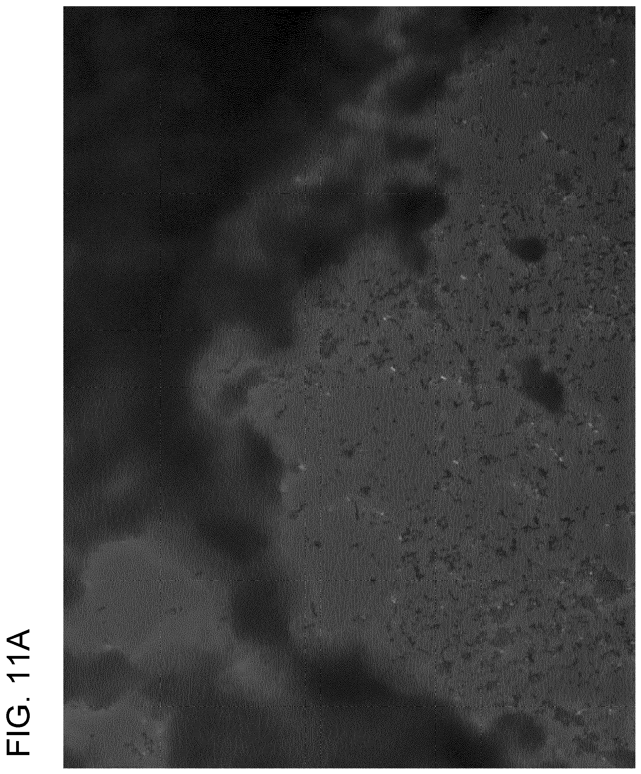

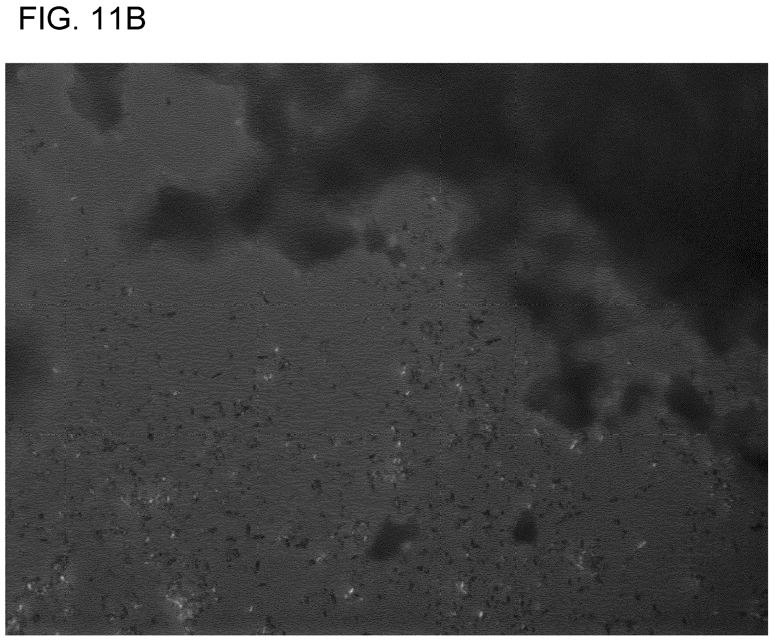

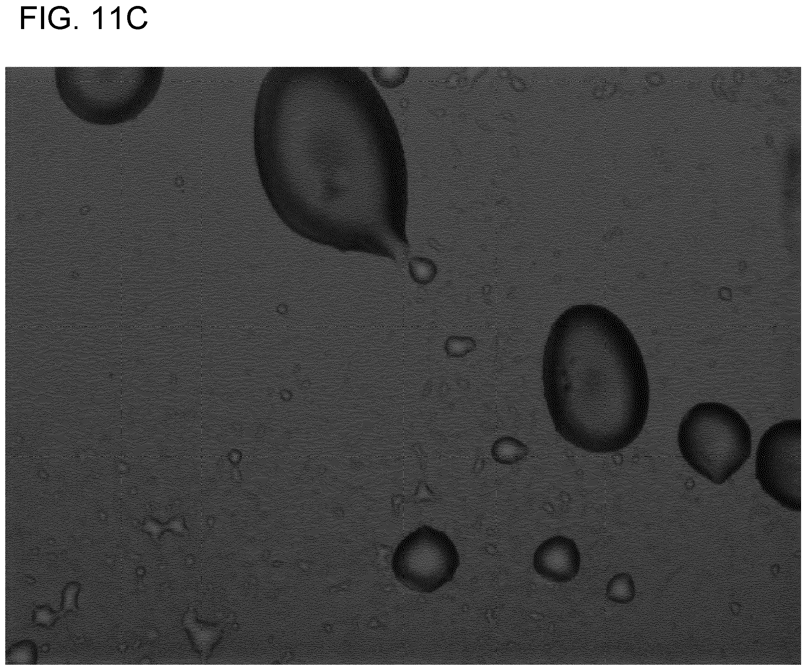

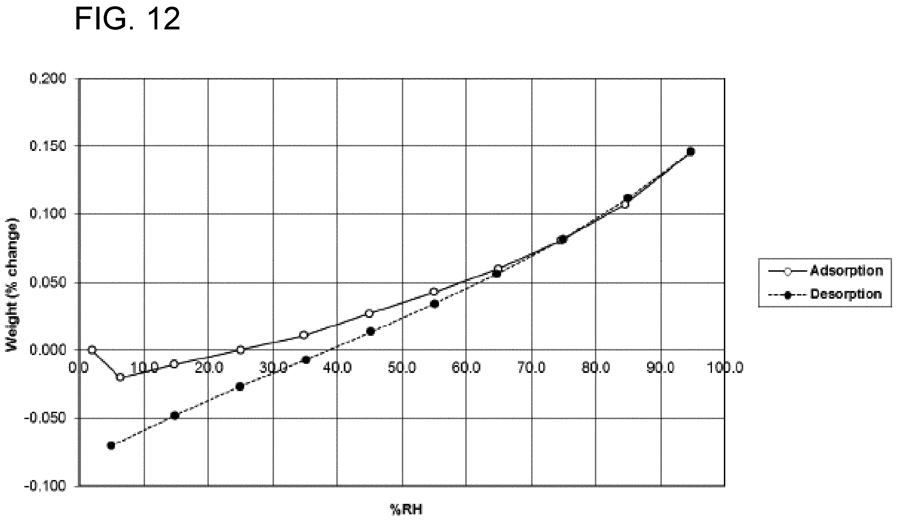

Sample 7179-08-01 exhibited a melting point of 192 °C and was analyzed by XRPD (FIG. 8), DSC (FIG. 9), TGA (FIG. 10), hot stage microscopy (FIG. 11), and DVS (FIG. 12). The DSC analysis revealed the presence of an endotherm at 186 °C, and TGA analysis revealed that there is a 0.6% weight loss up to 100 °C and an additional 0.6% weight loss up to 185 °C. Hot stage microscopy showed an onset of melting at 191.9 °C and completion of melting at 194.6 °C. DVS analysis revealed 0.0% weight change at 5% equilibration; 1.0% weight gain from 5 to 85% RH; 29.5% weight loss from 85 to 95% RH; and 25.4% weight loss from 95 to 5% RH. The XRPD pattern post DVS analysis is shown in FIG. 12.

### Preparation of Isobutyric Acid Addition Salt of Apomorphine (Reference)

A vessel was charged with 863.3 mg of apomorphine free base and 40 mL of diethyl ether to generate a slurry. A molar equivalent of isobutyric acid (0.300 mL) was added and the slurry was "seeded" with a small quantity of Isobutyrate Form A (7116-56-05). An additional 30 mL of diethyl ether was added, resulting in no visual changes. After approximately 10 minutes the vessel was charged with 5 mL of absolute ethanol and additional Isobutyrate Form A, resulting in a clear solution. The solution was treated with 50 mL of heptane and additional Isobutyrate Form A (7116-56-05). The resulting suspension was treated with an additional 10 mL of heptane and left to slurry for approximately 25 minutes at ambient. Limited material was observed to deposit above the solution. Upon scraping the material back into solution, it was noted that white solids readily formed upon evaporation. The sample was placed under nitrogen to reduce volume. Volume was reduced to about 20 mL of solution and the resulting white fines were recovered by filtration. The material was vacuum dried overnight at ambient.

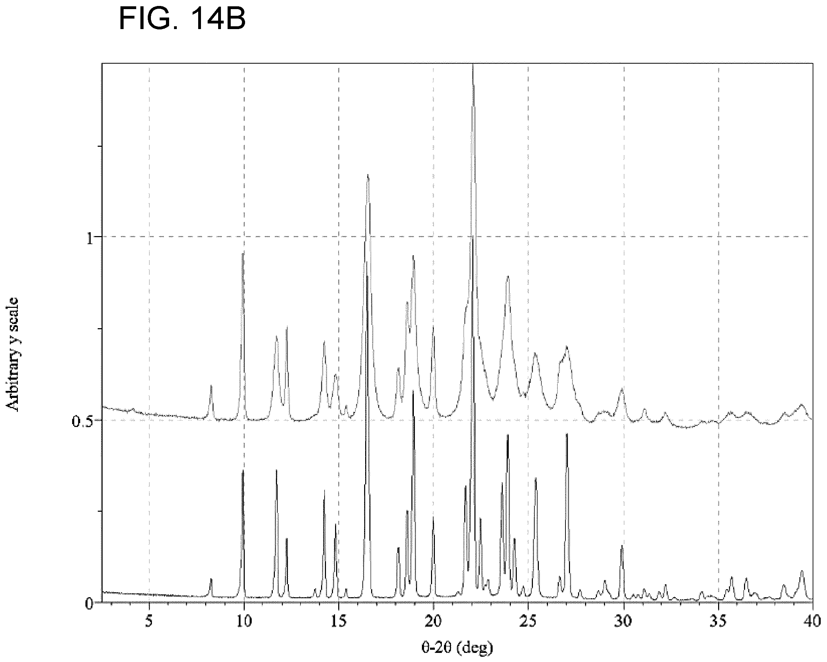

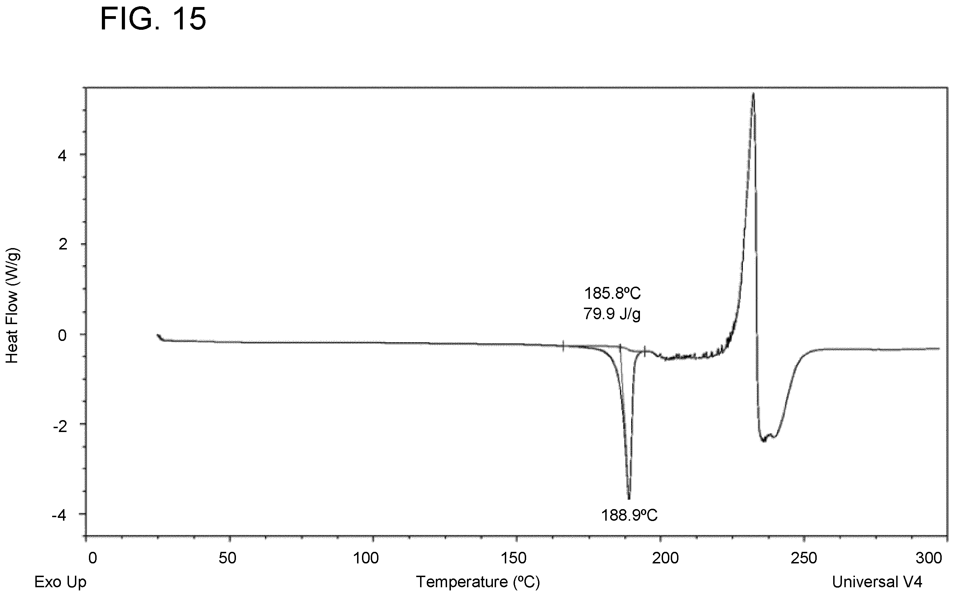

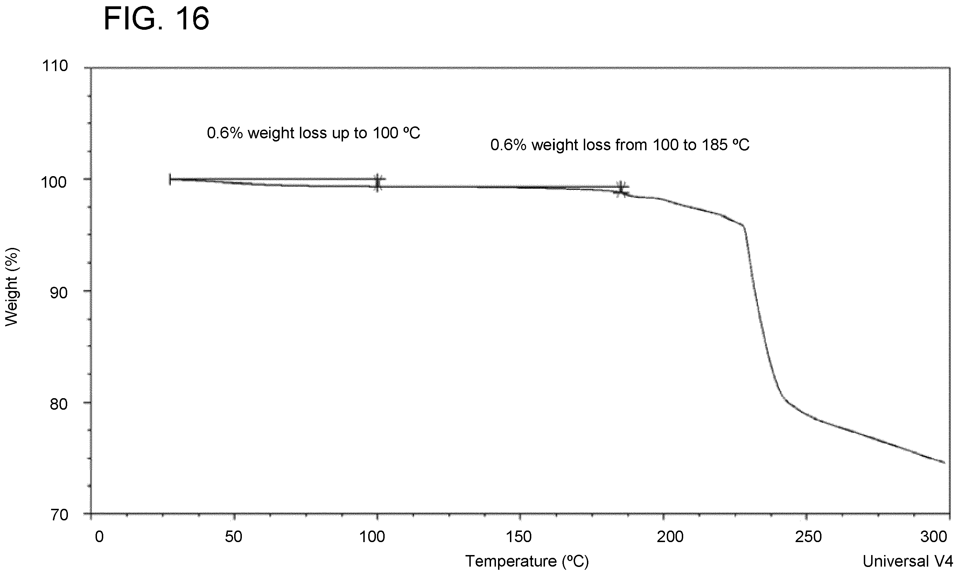

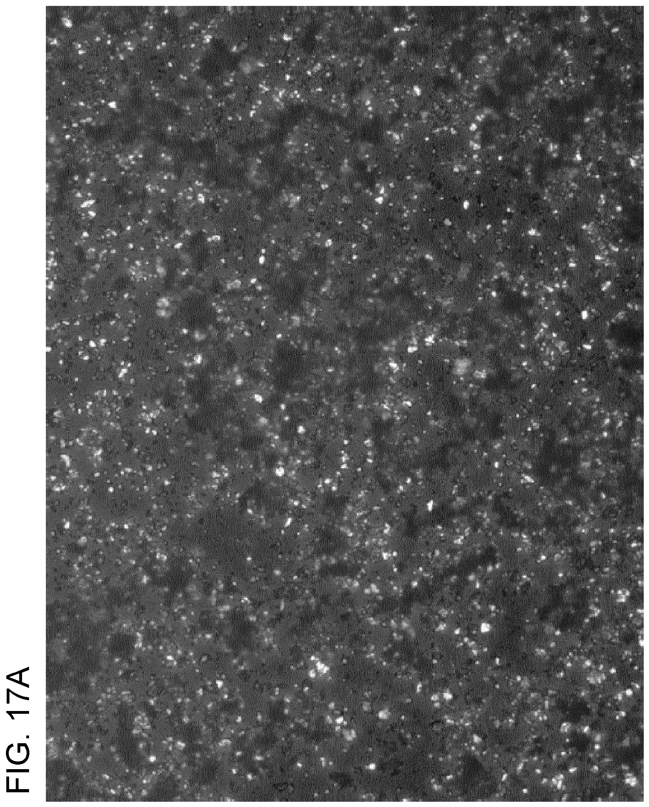

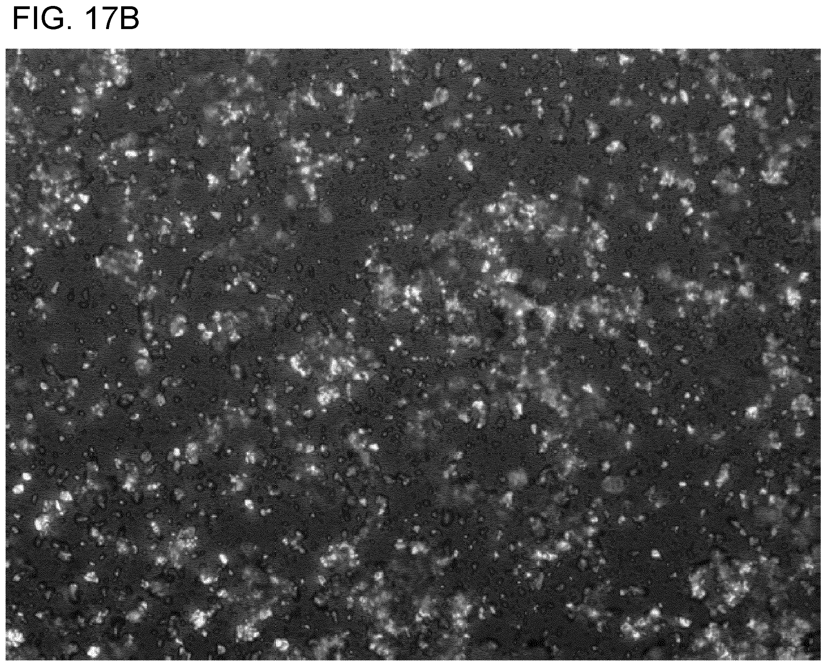

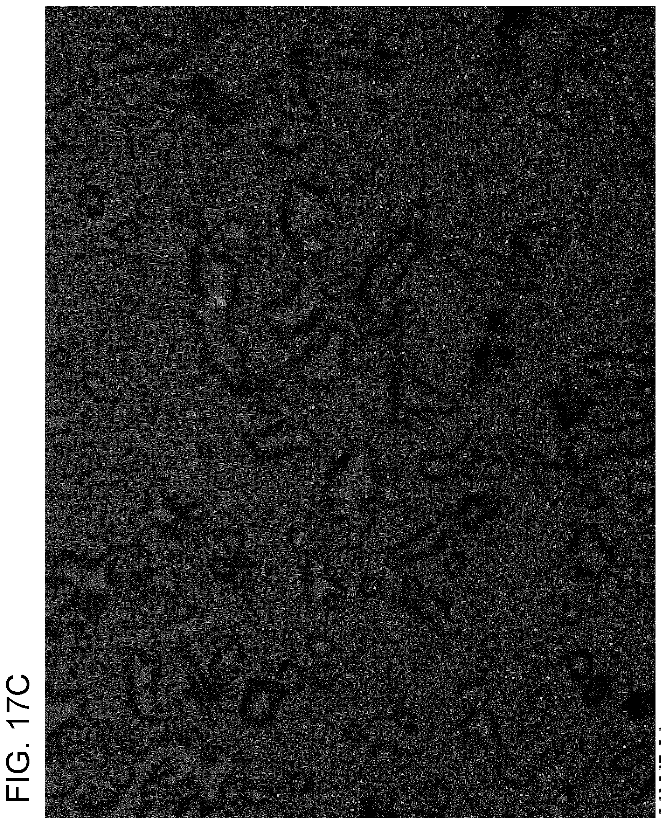

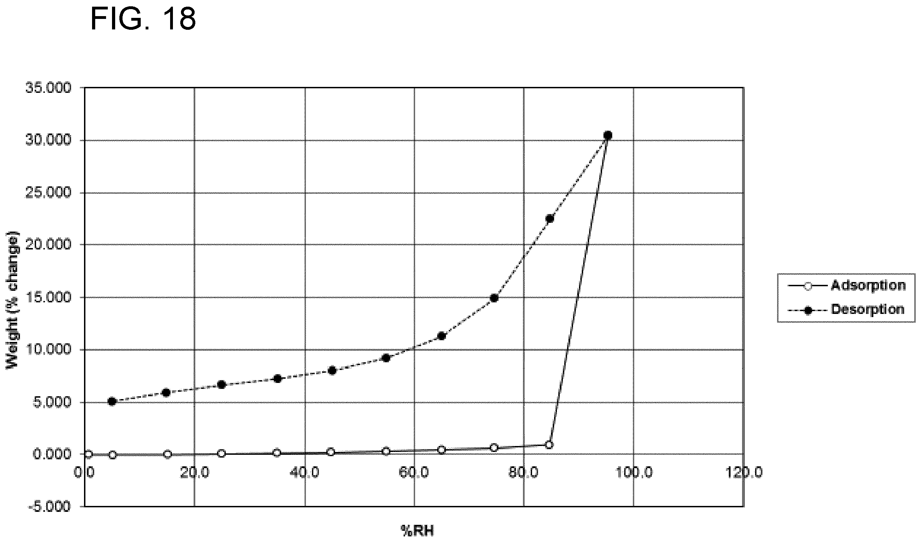

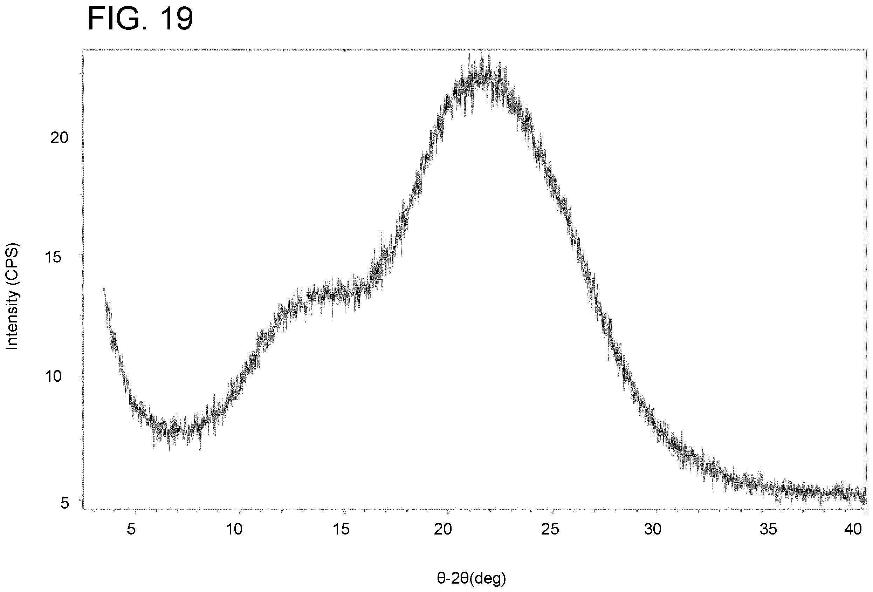

Sample 7179-07-01 exhibited a melting point of 128 °C and was analyzed by XRPD (FIG. 14), DSC (FIG. 15), TGA (FIG. 16), hot stage microscopy (FIG. 17), and DVS (FIG. 18). The DSC analysis revealed the presence of an endotherm at 144 °C, and TGA analysis revealed that there is a 0.5% weight loss up to 100 °C and 8.2% weight loss from 100 °C. Hot stage microscopy showed an onset of melting at 128.0 °C and completion of melting at 143.5 °C. DVS analysis revealed 0.0% weight change at 5% equilibration; 0.1% weight gain from 5 to 95% RH; and 0.2% weight loss from 95 to 5% RH. The XRPD pattern post DVS analysis is shown in FIG. 19.

### Example 3. Intrinsic Dissolution Rate Measurements

An intrinsic dissolution evaluation of four salts of acid addition salt of apomorphine (isobutyrate, sulfamate, glycolate, and hydrochloride) was conducted in two dissolution media: pH 4.5 acetate buffer and artificial saliva. The artificial saliva consisted of 30.4 g/L of sorbitol, 1.2 g/L potassium chloride, 0.86 g/L sodium chloride, 0.052 g/L of magnesium chloride hexahydrate, 0.112 g/L of calcium chloride, and 0.348 g/L of dipotassium phosphate (K₂HPO₃). Samples were quantitated using HPLC. Intrinsic dissolution experiments were carried out in duplicate for hydrochloride, isobutyrate, and sulfamate salts in both media. Intrinsic dissolution rate results are only available for a single experiment due to pellet fracturing and limited material.

Dissolution experiments were performed using a VanKel VK7010 dissolution tester equipped with a VK750D heater/circulator. A Wood's Apparatus (0.50 cm² sample surface area) as described in USP <1087> was used. Approximately 150 mg of acid addition salt of apomorphine salt were compressed with an applied load of approximately 1000 pounds for 1 minute in the Wood's apparatus using a hydraulic press. The dissolution medium was either pH 4.5 acetate buffer or artificial saliva. The medium (900 mL) was equilibrated to 37 °C ± 0.5 °C and degassed by sparging with helium for ca. 2 minutes at the start of each experiment. The disks were rotated at 75 rpm. Sampling of 1 mL aliquots was performed at the given timepoints using a 10 mL syringe equipped with a stainless steel cannula.

The sample aliquots were analyzed by HPLC for acid addition salt of apomorphine content. HPLC analyses were performed using an Agilent 1100 series liquid chromatograph equipped with a diode array or variable wavelength detector, degasser, quaternary pump, and an autosampler. The chromatographic column was a 4.6 x 50 mm Luna C18(2) column with 5 µm packing (Phenomenex). The column temperature was set to 25.0 °C, and the detector wavelength was 274 nm. The autosampler temperature was set to 5.0 °C. The injection volume was 25.0 µL. Mobile phase was 80:20 0.2% trifluoroacetic acid : acetonitrile.

As described in detail below, all salts dissolved faster in the pH 4.5 acetate buffer than the artificial saliva. Dissolution rates for apomorphine hydrochloride and apomorphine isobutyrate were significantly lower than the dissolution rates for apomorphine glycolate and apomorphine sulfamate. Available post-dissolution pellets were analyzed by XRPD, showing that no solid-form changes occurred during the experiments. The intrinsic dissolution rates (IDR) for the tested apomorphine salts in each tested medium are shown in Table 5.

**Table 5**

| **Apomorphine Salt** | **Dissolution Medium** | **IDR (mg/cm²/min)** |
|---|---|---|
| Hydrochloride | pH 4.5 acetate buffer | 2.643 |
| | Artificial saliva | 0.582 |
| Glycolate | pH 4.5 acetate buffer | 34.775 |
| | Artificial saliva | 10.862 |
| Isobutyrate | pH 4.5 acetate buffer | 1.576 |
| | Artificial saliva | 0.305 |
| Sulfamate | pH 4.5 acetate buffer | 19.598 |
| | Artificial saliva | 16.873 |

| | | |
|---|---|---|
| The sulfamate is according to the invention. The other salts are for reference. | | |

In Table 5, the IDR values for apomorphine hydrochloride, apomorphine sulfamate, and apomorphine isobutyrate are mean values for two replicated tests for each medium. Where rapid dissolution is desired, such as with sublingual administration for the treatment of Parkinson's disease, apomorphine glycolate and apomorphine sulfamate provide an advantage over, e.g., apomorphine hydrochloride. Rapid dissolution can be advantageous for producing a more rapid onset of action following, e.g., sublingual or buccal administration.

### Apomorphine Hydrochloride (Reference)

Intrinsic dissolution experiments were conducted in duplicate for apomorphine hydrochloride salt in pH 4.5 acetate buffer and artificial saliva. Aliquots (1 mL) were drawn from the dissolution vessel at time intervals indicated in Table 6, transferred to an HPLC vial, and the dissolution values (mass per unit area) of the apomorphine salt was determined for each aliquot (see Table 6). The determined dissolution values were plotted against the time intervals, and the slope of the linear curve was determined. This slope corresponds to the calculated intrinsic dissolution rate (IDR) for the apomorphine salt. The intrinsic dissolution data for apomorphine hydrochloride are listed in Table 6.

**Table 6**

| **Intrinsic Dissolution of Apomorphine Hydrochloride** | | | | | |
|---|---|---|---|---|---|
| **Dissolution in pH 4.5 Acetate Buffer (mg/cm²)** | | | **Dissolution in Artificial Saliva (mg/cm²)** | | |
| Time (min) | Test 1 | Test 2 | Time (min) | Test 1 | Test 2 |
| 1 | 2.169 | 2.520 | 5 | 3.064 | 3.429 |
| 3 | 7.620 | 6.919 | 10 | 6.064 | 6.704 |
| 5 | 11.873 | 13.670 | 15 | 8.379 | 9.102 |
| 10 | 25.544 | 24.811 | 20 | 11.502 | 12.455 |
| 15 | 40.377 | 37.495 | 25 | 15.043 | 14.655 |
| 20 | 53.844 | 51.425 | 30 | 18.250 | 17.510 |
| IDR (mg/cm²/min) | 2.736 | 2.551 | IDR (mg/cm²/min) | 0.606 | 0.558 |
| R² | 0.999 | 0.999 | R² | 0.997 | 0.996 |
| Mean IDR (mg/cm²/min) | 2.643 | | Mean IDR (mg/cm²/min) | 0.582 | |

After the dissolution study, remaining pellets were removed from the media and tested for any form changes by X-ray powder diffraction (XPRD), as described in Example 2. The post-dissolution XPRD confirms that no form changes occurred during the dissolution experiment.

### Apomorphine Glycolate (Reference)

Intrinsic dissolution experiments were conducted in duplicate for apomorphine glycolate salt in pH 4.5 acetate buffer and artificial saliva. Aliquots (1 mL) were drawn from the dissolution vessel at time intervals indicated in Table 7, transferred to an HPLC vial, and the dissolution values (mass per unit area) of the apomorphine salt was determined for each aliquot (see Table 7). The determined dissolution values were plotted against the time intervals, and the slope of the linear curve was determined. This slope corresponds to the calculated intrinsic dissolution rate (IDR) for the apomorphine salt. The intrinsic dissolution data for apomorphine glycolate are listed in Table 7.

**Table 7**

| **Intrinsic Dissolution of Apomorphine Glycolate** | | | |
|---|---|---|---|
| **Time (min)** | **Dissolution in pH 4.5 Acetate Buffer (mg/cm²)** | **Time (min)** | **Dissolution in Artificial Saliva (mg/cm²)** |
| 1 | 14.548 | - | - |
| 3 | 38.801 | 3 | 42.192 |
| 3.5 | 51.432 | 3.5 | 47.686 |
| 4 | 72.492 | 4 | 53.878 |
| 4.5 | 89.739 | 4.5 | 58.232 |
| IDR (mg/cm²/min) | 34.775 | IDR (mg/cm²/min) | 10.862 |
| R² | 0.907 | R² | 0.996 |

After the dissolution study, remaining pellets were removed from the media and tested for any form changes by X-ray powder diffraction (XPRD), as described in Example 2. The post-dissolution XPRD confirms that no form changes occurred during the dissolution experiment.

### Apomorphine Isobutyrate (Reference)

Intrinsic dissolution experiments were conducted in duplicate for apomorphine isobutyrate salt in pH 4.5 acetate buffer and artificial saliva. Aliquots (1 mL) were drawn from the dissolution vessel at time intervals indicated in Table 8, transferred to an HPLC vial, and the dissolution values (mass per unit area) of the apomorphine salt was determined for each aliquot (see Table 8). The determined dissolution values were plotted against the time intervals, and the slope of the linear curve was determined. This slope corresponds to the calculated intrinsic dissolution rate (IDR) for the apomorphine salt. The intrinsic dissolution data for apomorphine isobutyrate are listed in Table 8.

**Table 8**

| Intrinsic Dissolution of Apomorphine Isobutyrate | | | | | |
|---|---|---|---|---|---|
| Dissolution in pH 4.5 Acetate Buffer (mg/cm²) | | | Dissolution in Artificial Saliva (mg/cm²) | | |
| Time (min) | Test 1 | Test 2 | Time (min) | Test 1 | Test 2 |
| 1 | 6.967 | 7.913 | 5 | 1.892 | 2.501 |
| 3 | 13.325 | 15.397 | 10 | 3.834 | 4.966 |
| 5 | 21.552 | 25.373 | 15 | 4.868 | 6.235 |
| 10 | 29.269 | 33.476 | 20 | 6.503 | 7.959 |
| 15 | 38.423 | 37.892 | 25 | 7.610 | 9.847 |
| 20 | 46.769 | 46.691 | 30 | 8.858 | 11.032 |
| IDR (mg/cm²/min) | 1.612 | 1.540 | IDR (mg/cm²/min) | 0.273 | 0.337 |
| R² | 0.998 | 0.992 | R² | 0.993 | 0.991 |
| Mean IDR (mg/cm²/min) | 1.576 | | Mean IDR (mg/cm²/min) | 0.305 | |

After the dissolution study, remaining pellets were removed from the media and tested for any form changes by X-ray powder diffraction (XPRD), as described in Example 2. The post-dissolution XPRD confirms that no form changes occurred during the dissolution experiment.

### Apomorphine Sulfamate

Intrinsic dissolution experiments were conducted in duplicate for apomorphine sulfamate salt in pH 4.5 acetate buffer and artificial saliva. Aliquots (1 mL) were drawn from the dissolution vessel at time intervals indicated in Table 9, transferred to an HPLC vial, and the dissolution values (mass per unit area) of the apomorphine salt was determined for each aliquot (see Table 9). The determined dissolution values were plotted against the time intervals, and the slope of the linear curve was determined. This slope corresponds to the calculated intrinsic dissolution rate (IDR) for the apomorphine salt. The intrinsic dissolution data for apomorphine sulfamate are listed in Table 9.

**Table 9**

| Intrinsic Dissolution of Apomorphine Sulfamate | | | | | |
|---|---|---|---|---|---|
| Dissolution in pH 4.5 Acetate Buffer (mg/cm²) | | | Dissolution in Artificial Saliva (mg/cm²) | | |
| Time (min) | Test 1 | Test 2 | Time (min) | Test 1 | Test 2 |
| 1 | 12.015 | 10.377 | 5 | 10.745 | 10.577 |
| 3 | 20.468 | 29.889 | 10 | 16.867 | 17.208 |
| 5 | 29.803 | 36.346 | 15 | 28.653 | 27.680 |
| 10 | 37.717 | 46.685 | 20 | 34.629 | 35.306 |
| 15 | 49.192 | 59.176 | 25 | 40.740 | 52.528 |
| 20 | 53.887 | 67.234 | 30 | 48.782 | 52.414 |
| IDR (mg/cm²/min) | 17.340 | 21.856 | IDR (mg/cm²/min) | 15.301 | 18.444 |
| R² | 0.993 | 0.980 | R² | 0.990 | 0.964 |
| Mean IDR (mg/cm²/min) | 19.598 | | Mean IDR (mg/cm²/min) | 16.873 | |

After the dissolution study, remaining pellets were removed from the media and tested for any form changes by X-ray powder diffraction (XPRD), as described in Example 2. The post-dissolution XPRD confirms that no form changes occurred during the dissolution experiment.

### Example 4. Compression Study

A compression study was performed to confirm that compression does not result in the salt form change. Quantities of apomorphine salt (*ca.* 150 mg for the HCl salt, *ca.* 70 mg for other salts due to limited availability of material) were weighed into the die cavity of a Woods apparatus and then compressed for 1 minute using an applied load of approximately 1000 pounds. The pressed pellets were removed from the die cavity and analyzed by XRPD. The XRPD results are shown in FIGs. 1, 2B, 8B, and 14B. In these figures, compared to the respective starting materials. The resulting XRPD patterns show that no form changes occurred during the compressions. Some reduction of crystallinity was observed for apomorphine hydrochloride, apomorphine sulfamate, and apomorphine isobutyrate. The sulfamate is according to the invention. The other salts are for reference.

## Claims

1. An acid addition salt of apomorphine, wherein the acid is sulfamic acid.

2. A solid crystalline form of the acid addition salt of claim 1, wherein the acid addition salt of apomorphine is apomorphine sulfamate, and the crystalline form is **characterized by** an X-ray powder diffraction (XRPD) pattern comprising one or more peaks selected from peaks at diffraction angles 2θ (°) of 9.9±0.2, 11.7±0.2, 14.2±0.2, 14.8±0.2, 16.5±0.2, 18.6±0.2, 18.9±0.2, 21.7±0.2, 22.1±0.2, 22.4±0.2, 23.6±0.2, 23.9±0.2, 25.4±0.2, and 27.0±0.2.

3. The solid crystalline form of claim 2, wherein the X-ray powder diffraction (XRPD) pattern comprises one or more peaks selected from peaks at diffraction angles 2θ (°) of 9.9±0.2, 11.7±0.2, 16.5±0.2, 18.9±0.2, 22.1±0.2, 23.9±0.2, and 27.0±0.2.

4. The solid crystalline form of claim 2, wherein the X-ray powder diffraction (XRPD) pattern comprises peaks at diffraction angles 2θ (°) of 9.9±0.2, 11.7±0.2, 16.5±0.2, 18.9±0.2, 22.1±0.2, 23.9±0.2, and 27.0±0.2.

5. The solid crystalline form of claim 2, wherein the X-ray powder diffraction (XRPD) pattern comprises peaks at diffraction angles 2θ (°) of 9.9±0.2, 11.7±0.2, 14.2±0.2, 14.8±0.2, 16.5±0.2, 18.6±0.2, 18.9±0.2, 21.7±0.2, 22.1±0.2, 22.4±0.2, 23.6±0.2, 23.9±0.2, 25.4±0.2, and 27.0±0.2.

6. A pharmaceutical composition comprising the acid addition salt of claim 1 or the solid crystalline form of any one of claims 2 to 5 and a pharmaceutically acceptable excipient.

7. The pharmaceutical composition of claim 6, wherein the pharmaceutical composition is a unit dosage form selected from a film, lozenge, troche, tablet, cream, gel, ointment, liquid solution or suspension, powder, or capsule, preferably wherein the unit dosage form is a film.

8. The pharmaceutical composition of claim 6 or 7, formulated for sublingual or buccal administration.

9. The acid addition salt of claim 1, the solid crystalline form of any one of claims 2 to 5, or the pharmaceutical composition of any one of claims 6 to 8 for use in the treatment of Parkinson's disease.

10. The acid addition salt, the solid crystalline form, or the pharmaceutical composition for use of claim 9, wherein the use is the use in the treatment of an "off" episode.

11. The acid addition salt, the solid crystalline form, or the pharmaceutical composition for use of claim 9, wherein the use is the use in the treatment of an acute or intermittent "OFF" episode associated with Parkinson's disease.

12. The acid addition salt, the solid crystalline form, or the pharmaceutical composition for use of claim 9, wherein the use is the use in switching a subject "on" from an "off" state.

13. The acid addition salt of claim 1, the solid crystalline form of any one of claims 2 to 5, or the pharmaceutical composition of any one of claims 6 to 8 for use in the treatment of restless leg syndrome.

14. The acid addition salt of claim 1, the solid crystalline form of claim 2 to 5, or the pharmaceutical composition of any one of claims 6 to 8 for use in the treatment of sexual dysfunction.

15. A method of preparing the acid addition salt of apomorphine of claim 1, the method comprising reacting free-base apomorphine with sulfamic acid.

## Patentansprüche

1. Ein Säureadditionssalz von Apomorphin, wobei die Säure Sulfaminsäure ist.

2. Eine feste kristalline Form des Säureadditionssalzes gemäß Anspruch 1, wobei das Säureadditionssalz von Apomorphin Apomorphinsulfamat ist und die kristalline Form durch ein Röntgenpulverbeugungsmuster (XRPD) gekennzeichnet ist, das einen oder mehrere Peaks umfasst, ausgewählt aus Peaks bei Beugungswinkeln 2θ (°) von 9,9±0,2, 11,7±0,2, 14,2±0,2, 14,8±0,2, 16,5±0,2, 18,6±0,2, 18,9±0,2, 21,7±0,2, 22,1±0,2, 22,4±0,2, 23,6±0,2, 23,9±0,2, 25,4±0,2 und 27,0±0,2.

3. Die feste kristalline Form gemäß Anspruch 2, wobei das Röntgenpulverbeugungsmuster (XRPD) einen oder mehrere Peaks umfasst, ausgewählt aus Peaks bei Beugungswinkeln 2θ (°) von 9,9±0,2, 11,7±0,2, 16,5±0,2, 18,9±0,2, 22,1±0,2, 23,9±0,2 und 27,0±0,2.

4. Die feste kristalline Form gemäß Anspruch 2, wobei das Röntgenpulverbeugungsmuster (XRPD) Peaks bei Beugungswinkeln 2θ (°) von 9,9±0,2, 11,7±0,2, 16,5±0,2, 18,9±0,2, 22,1±0,2, 23,9±0,2 und 27,0±0,2 umfasst.

5. Die feste kristalline Form gemäß Anspruch 2, wobei das Röntgenpulverbeugungsmuster (XRPD) Peaks bei Beugungswinkeln 2θ (°) von 9,9±0,2, 11,7±0,2, 14,2±0,2, 14,8±0,2, 16,5±0,2, 18,6±0,2, 18,9±0,2, 21,7±0,2, 22,1±0,2, 22,4±0,2, 23,6±0,2, 23,9±0,2, 25,4±0,2 und 27,0±0,2 umfasst.

6. Ein Arzneimittel, umfassend das Säureadditionssalz gemäß Anspruch 1 oder die feste kristalline Form gemäß einem der Ansprüche 2 bis 5 und einen pharmazeutisch verträglichen Exzipienten.

7. Das Arzneimittel gemäß Anspruch 6, wobei das Arzneimittel eine Einheits-Dosierungsform ist, ausgewählt aus einem Film, einer Lutschtablette, einer Lutschpastille, einer Tablette, einer Creme, einem Gel, einer Salbe, einer flüssigen Lösung oder Suspension, einem Pulver oder einer Kapsel, wobei die Einheits-Dosierungsform vorzugsweise ein Film ist.

8. Das Arzneimittel gemäß Anspruch 6 oder 7, das zur sublingualen oder bukkalen Verabreichung formuliert ist.

9. Das Säureadditionssalz gemäß Anspruch 1, die feste kristalline Form gemäß einem der Ansprüche 2 bis 5 oder das Arzneimittel gemäß einem der Ansprüche 6 bis 8 zur Verwendung bei der Behandlung der Parkinson-Krankheit.

10. Das Säureadditionssalz, die feste kristalline Form oder das Arzneimittel zur Verwendung gemäß Anspruch 9, wobei die Verwendung die Verwendung zur Behandlung einer "Off"-Episode ist.

11. Das Säureadditionssalz, die feste kristalline Form oder das Arzneimittel zur Verwendung gemäß Anspruch 9, wobei die Verwendung die Verwendung zur Behandlung einer akuten oder intermittierenden "OFF"-Episode im Zusammenhang mit der Parkinson-Krankheit ist.

12. Das Säureadditionssalz, die feste kristalline Form oder das Arzneimittel zur Verwendung gemäß Anspruch 9, wobei die Verwendung die Verwendung zum Umschalten eines Patienten aus einem "OFF"-Zustand in einen "ON"-Zustand ist.

13. Das Säureadditionssalz gemäß Anspruch 1, die feste kristalline Form gemäß einem der Ansprüche 2 bis 5 oder das Arzneimittel gemäß einem der Ansprüche 6 bis 8 zur Verwendung bei der Behandlung des Restless-Leg-Syndroms.

14. Das Säureadditionssalz gemäß Anspruch 1, die feste kristalline Form gemäß Anspruch 2 bis 5 oder das Arzneimittel gemäß einem der Ansprüche 6 bis 8 zur Verwendung bei der Behandlung von sexueller Dysfunktion.

15. Ein Verfahren zur Herstellung des Säureadditionssalzes von Apomorphin gemäß Anspruch 1, wobei das Verfahren die Umsetzung von Apomorphin in freier Base mit Sulfaminsäure umfasst.

## Revendications

1. Sel d'addition d'acide d'apomorphine, dans lequel l'acide est de l'acide sulfamique.

2. Forme cristalline solide du sel d'addition d'acide selon la revendication 1, dans laquelle le sel d'addition d'acide d'apomorphine est de l'apomorphine sulfamate, et la forme cristalline est **caractérisée par** un motif de diffraction des rayons X sur poudres (XRPD) comprenant un ou plusieurs pics sélectionnés parmi des pics à des angles de diffraction 2θ (°) de 9,9±0,2, 11,7±0,2, 14,2±0,2, 14,8±0,2, 16,5±0,2, 18,6±0,2, 18,9±0,2, 21,7±0,2, 22,1±0,2, 22,4±0,2, 23,6±0,2, 23,9±0,2, 25,4±0,2 et 27,0±0,2.

3. Forme cristalline solide selon la revendication 2, dans laquelle le motif de diffraction des rayons X sur poudres (XRPD) comprend un ou plusieurs pics sélectionnés parmi des pics à des angles de diffraction 2θ (°) de 9,9±0,2, 11,7±0,2, 16,5±0,2, 18,9±0,2, 22,1±0,2, 23,9±0,2 et 27,0±0,2.

4. Forme cristalline solide selon la revendication 2, dans laquelle le motif de diffraction des rayons X sur poudres (XRPD) comprend des pics à des angles de diffraction 2θ (°) de 9,9±0,2, 11,7±0,2, 16,5±0,2, 18,9±0,2, 22,1±0,2, 23,9±0,2 et 27,0±0,2.

5. Forme cristalline solide selon la revendication 2, dans laquelle le motif de diffraction des rayons X sur poudres (XRPD) comprend des pics à des angles de diffraction 2θ (°) de 9,9±0,2, 11,7±0,2, 14,2±0,2, 14,8±0,2, 16,5±0,2, 18,6±0,2, 18,9±0,2, 21,7±0,2, 22,1±0,2, 22,4±0,2, 23,6±0,2, 23,9±0,2, 25,4±0,2 et 27,0±0,2.

6. Composition pharmaceutique comprenant le sel d'addition d'acide selon la revendication 1 ou la forme cristalline solide selon l'une quelconque des revendications 2 à 5 et un excipient pharmaceutiquement acceptable.

7. Composition pharmaceutique selon la revendication 6, dans laquelle la composition pharmaceutique est une forme de dosage unitaire sélectionnée parmi un film, pastille, tablette, comprimé, crème, gel, pommade, solution ou suspension liquide, poudre ou gélule, de préférence dans laquelle la forme de dosage unitaire est un film.

8. Composition pharmaceutique selon la revendication 6 ou 7, formulée pour une administration sublinguale ou buccale.

9. Sel d'addition d'acide selon la revendication 1, forme cristalline solide selon l'une quelconque des revendications 2 à 5, ou composition pharmaceutique selon l'une quelconque des revendications 6 à 8 pour son utilisation dans le traitement de la maladie de Parkinson.

10. Sel d'addition d'acide, forme cristalline solide ou composition pharmaceutique pour son utilisation selon la revendication 9, dans lequel l'utilisation est l'utilisation dans le traitement d'un épisode « off » (réapparition des symptômes).

11. Sel d'addition d'acide, forme cristalline solide, ou composition pharmaceutique pour son utilisation selon la revendication 9, dans lequel l'utilisation est l'utilisation dans le traitement d'un épisode « OFF » aigu ou intermittent associé à la maladie de Parkinson.

12. Sel d'addition d'acide, forme cristalline solide ou composition pharmaceutique pour son utilisation selon la revendication 9, dans lequel l'utilisation est l'utilisation pour commuter un sujet en état « on » depuis un état « off ».

13. Sel d'addition d'acide selon la revendication 1, forme cristalline solide selon l'une quelconque des revendications 2 à 5, ou composition pharmaceutique selon l'une quelconque des revendications 6 à 8 pour son utilisation dans le traitement du syndrome des jambes agitées.

14. Sel d'addition d'acide selon la revendication 1, forme cristalline solide selon les revendications 2 à 5, ou composition pharmaceutique selon l'une quelconque des revendications 6 à 8 pour son utilisation dans le traitement de dysfonctionnement sexuel.

15. Méthode de préparation du sel d'addition d'acide d'apomorphine selon la revendication 1, la méthode comprenant la réaction de l'apomorphine à base libre avec de l'acide sulfamique.
